(19) Europäisches Patentamt

European Patent Office

Office européen des brevets

(11) **EP 1 450 288 A2**

(12) **EUROPEAN PATENT APPLICATION**

(43) Date of publication:
**25.08.2004 Bulletin 2004/35**

(51) Int Cl.⁷: **G06F 19/00**

(21) Application number: **04447012.8**

(22) Date of filing: **15.01.2004**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **15.01.2003 EP 03447011**

(71) Applicant: **Applied Maths BVBA**
**9830 Sint-Martens-Latem (BE)**

(72) Inventors:
• **Vauterin, Luc**
  **9831 Deurle (BE)**
• **Vauterin, Paul**
  **9000 Gent (BE)**

(74) Representative:
**Brants, Johan Philippe Emile et al**
**De Clercq, Brants & Partners cv**
**Edgard Gevaertdreef 10a**
**9830 Sint-Martens-Latem (BE)**

(54) **A method for obtaining consensus classifications and identifications by combining data from different experiments**

(57)     The present invention relates to methods for producing accurate consensus classifications of organisms by combining similarity matrices. It further relates to an apparatus and computer program therefor.

# Figure 1-B

| | G1 | G2 | G3 | G4 |
|---|---|---|---|---|
| **G1** | 100 | | | |
| **G2** | 68 | 100 | | |
| **G3** | 77 | 96 | 100 | |
| **G4** | 97 | 85 | 72 | 100 |

**Description**

**BACKGROUND TO THE INVENTION**

Classification techniques have been used in the field of biology to determine relationships and patterns in large sets of data. A means for placing an unidentified organism into a classification group which reflects its genotype is important to obtain an understanding of the organism and its features, particularly in the treatment and study of disease and in the assessment of biodiversity. Information which identifies the closest known relation to an unknown disease-causing bacterial or viral strain would enable a physician to prescribe the most appropriate course of treatment based on such knowledge. Similarly, information which could, for example, enable a group of patients suffering from a disorder that has several genetic and phenotypical traits to be catagorised into nearest-relation groups would enable subpopulations of patients to receive the appropriate treatment and/or enable a research scientist to discern the most appropriate set of genes and associated biochemical pathways for further study. More particularly, the study of infections and epidemic diseases, for example, in hospitals or caused by distributed food or contaminated water, can benefit from adequate classification of the causative agents, in a way that the sources of an outbreak or infection can be traced and eradicated. The development of DNA chips and microarrays has increased the amount of data available for analysis, and increased the need for methods that can rapidly and reliably analyse raw experimental data.

Classification techniques have been described in the art. PCT patent application numbers WO 01/20536 and WO 01/73602 disclose methods for producing hierarchical clusterings from large sets of biological data. PCT patent application WO 01/45026 discloses a method for displaying data resulting from a consensus classification analysis in a visually comprehensible format.

While methods to determine classifications have been developed to deal with the processing of large amounts of experimental data, the problem remains with the accuracy of the classifications. Present methods do not take account of the quality of the experimental data from which classifications are made, and hence the classifications so-produced are distorted, leading to incorrect analyses which may seriously affect the course of a treatment regimen. A further problem with conventional classification methods is that they are unable to provide a classification when data is missing, or they ignore the fact that an item of data is missing and distort the consensus classification so produced. Distorted and incorrect consensus classifications have serious implications for medical research and for the treatment of diseases. Thus there are time/cost and health benefits in finding a method which can produce accurate consensus classifications.

**AIMS OF THE INVENTION**

It is an aim of the present invention to provide a method suitable for producing accurate classifications and which overcomes the limitations of the prior art. It is further an aim of the present invention to provide an apparatus for producing accurate classifications and which overcomes the limitations of the prior art.

**SUMMARY OF THE INVENTION**

One embodiment of the invention is a method suitable for producing a consensus classification of organisms using the data derived from two or more experiments performed on said organisms or samples thereof comprising the steps of:

i) obtaining similarity matrices from the said data,
ii) producing a composite similarity matrix that is a function of said similarity matrices, and
iii) producing a consensus classification from said composite similarity matrix.

Another embodiment of the invention is a method as described above wherein the function of step ii) comprises averaging the corresponding elements of said similarity matrices.

Another embodiment of the invention is a method as described above wherein each similarity matrix is weighted according the number of experimental characters used to calculate said matrix, to arrive at the average.

Another embodiment of the invention is a method as described above wherein each similarity matrix is weighted by a user defined value to arrive at the average.

Another embodiment of the invention is a method as described above, wherein said experiments produce product size or retention time results, and wherein the each element of each similarity matrix is weighted according to the number of bands or features associated with that element, to arrive at the average.

Another embodiment of the invention is a method as described above wherein said experiments are any of electrophoresis, high performance liquid chromatography, gas chromatography, capillary electrophoresis, chromatography, thin-layer chromatography, and/or mass spectrometry.

Another embodiment of the invention is a method as described above wherein the function of step ii) comprises the steps of:

a) linearising said similarity data matrices,
b) averaging the corresponding elements of said linearised similarity matrices of step a)

Another embodiment of the invention is a method as described above wherein step a) comprises the minimisation of equations:

$$\sum_{i=1}^{p}\sum_{j=1}^{i-1}\left(\hat{d}_{k,ij}-f_k(D_{ij})\right)^2 \ , \ \forall k$$

$$\sum_{i=1}^{p}\sum_{j=1}^{i-1}\left(D_{ij}-g_k(\hat{d}_{k,ij})\right)^2 \ , \ \forall k$$

wherein $p$ is the number or organisms, samples or genotypes, wherein each technique $k$ results in a matrix of pairwise distance values, so that the distance value obtained between organism i and j from technique k is given by $d_{k,ij}$,

wherein $\hat{d}_{k,ij} = \dfrac{d_{k,ij}}{S_k}$ with

$$S_k = \frac{2}{(p-1)(p-2)}\sum_{i=1}^{p}\sum_{j=1}^{i-1}d_{k,ij} \ ,$$

wherein the consensus distance matrix $D_{ij}$ is considered as the unknown true universal distance scale and wherein the goal is to search the consensus distances $D_{ij}$, $g_k$ and $f_k$ so that $d_{k,ij} \cong f_k(D_{ij})$ and $D_{ij} \cong g_k(d_{k,ij})$ hold as true as possible.

Another embodiment of the invention is an apparatus suitable for performing the methods as described above.

Another embodiment of the invention is a computer program comprising a computing routine, stored on a computer readable medium suitable for producing a consensus classification of organisms using the data derived from two or more experiments performed on said organisms or samples thereof according to the methods as described above.

Another embodiment of the invention is a device suitable for producing a consensus classification of organisms using the data derived from two or more experiments performed on said organisms or samples thereof according to the methods as described above.

## DETAILED DESCRIPTION OF THE INVENTION

As used herein the term, "classification" refers to the classification of organisms, or of the data from experiments performed on said organisms or samples thereof.

The term "consensus classification" refers to a classification of organisms, or of the data from experiments performed on said organisms or samples thereof, wherein the data is derived from two or more techniques, and wherein said techniques produce one or more data types.

A consensus classification may show the degree of relationship between organisms or samples thereof. Consensus classification may indicate the hierarchical ordering within a set of organisms or samples thereof. Consensus classifications may provide information necessary to construct a dendrogram or grouping by means other than cluster analysis.

By "organism" herein is meant any animal or plant, any cell, bacterium, yeast, phage, virus or prion; it includes organisms of any genus, species, sub-type, biotype, phenotype or genotype.

By "sample" herein is meant a portion which is intended to represent the whole. Non-limiting examples of samples are environmental samples such as soil, water, or clinical samples such as blood, sputum, stool, urine. Samples may contain many organisms as defined above. Samples may comprise extracts of organisms such as their DNA, proteins, glycoproteins, or other quantifiable substances.

In one embodiment of the present invention, data derived from experiments is classified according to the "data type" that is produced by the result of the experiment.

In one embodiment of the present invention, the data type is one or more "binary characters". By "binary characters" is meant the result(s) from an experiment is one of two outcomes, said result recordable as a binary character. For example, the outcome of the experiment might be either "present" or "absent", "positive" or "negative", "high" or "low", "dark" or "bright", recordable as a binary 1 or 0.

In another embodiment of the present invention, the data type is one or more "continuous-scale characters". By "continuous-scale characters " is meant the result(s) from an experiment is a value which reflects a magnitude, said result recordable as a continuous-scale character such as, for example, a decimal number between -100 and +100. This category includes, but is not limited to, data from experiments to measure concentrations, kinetic properties, intensities, etc.

In another embodiment of the present invention, the data type is one or more "multistate or categorical characters". By "multistate or categorical characters" is meant the result(s) from an experiment is a state or category, said result recordable as a state or category name-tag. This category includes, but is not limited to, for example data from experiments which result in a colour (*e.g.* category names red, blue, yellow, black, green), a shape (*e.g.* category names cube, sphere, cylinder), but also sequence-related genotypic properties such as genotypes resulting from Multilocus Sequence Typing (MLST) ,Variable Number of Tandem Repeats (VNTR) typing, Microsatelite analysis, DNA conformational structure analysis (Single Strand Conformational Polymorphism and heteroduplex electrophoresis), Single Nucleotide Polymorphism (SNP) analysis, etc. Binary characters may be interchangeable with multistate or categorical characters when the result of an experiment is one of two categories.

In another embodiment of the present invention, the data type is one ore more "product size or retention time characters". By "product size or retention time characters" is meant the result(s) from an experiment is the magnitude of one property recorded as a function of the magnitude of a second property, recordable as a character. This category includes, but is not limited to, data from experiments which result in an electrophoresis gel, a high performance liquid chromatography (HPLC) chromatogram, a gas chromatogram, a capillary electrophoresis chromatogram, a paper chromatogram, a thin-layer chromatogram, mass spectrometry techniques such as MALDI-TOF.

In another embodiment of the present invention, the data type is one or more "sequence characters ". By "sequence characters" is meant the result(s) from an experiment is a sequence of characters corresponding to, for example, DNA, RNA and/or amino acid sequences, recordable as a sequence of characters.

In one embodiment of the present invention, characters of data derived from experimental tests, observations or recordings taken from samples of organisms (e.g. intra and/or extracellular material from one of more organisms) are collected.

In one embodiment of the present invention, said data is recorded as an "experimental data matrix".

As used herein an "experiment" refers to an experimental action or set of experimental actions which leads to an observation or a set of observations that can be recorded as a dimensional array of measurements for a single organism, sample or genotype. Non-limiting examples of experiments include measuring cell length, determining an antibiotic resistance profile, incubating a microtiter plate to measure 96 enzymatic activities, running an RFLP electrophoresis pattern, a two-dimensional gel, obtaining a DNA sequence.

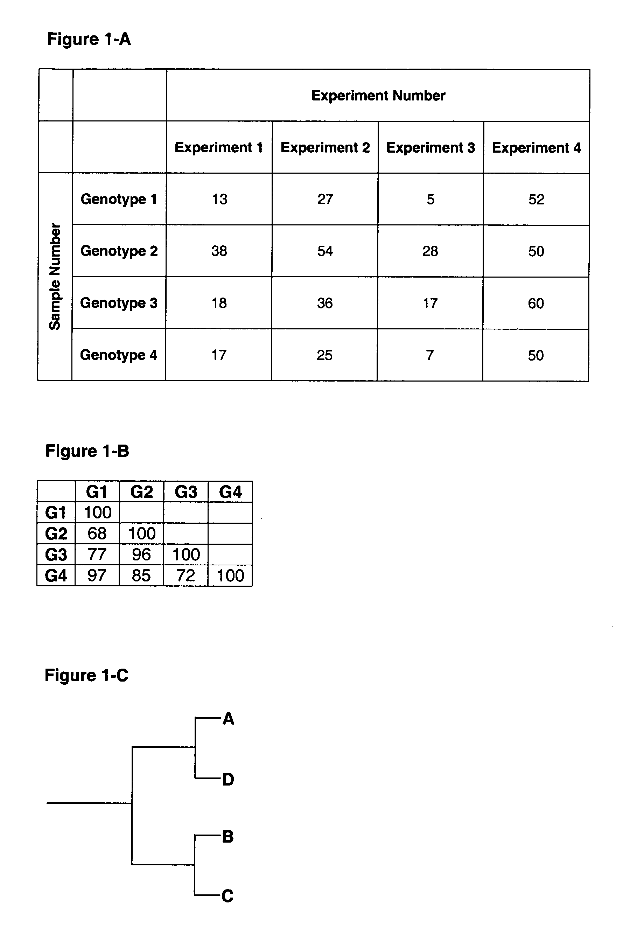

As used herein an "experimental data matrix" means a set of results from one or more individual experiments of the same data type. A non-limiting example of an experimental data matrix is given in Figure 1-A, wherein four different experiments which produce results of the same data type (experiments 1 to 4) have been performed on samples from four different organisms (genotype 1 to 4). While the results are presented as a matrix in Figure 1-A, the term "matrix", as used herein, does not limit the data to the matrix format; data may be recorded or presented in any format. Non-limiting examples of data formats include dimensional arrays, lists, coded data, data.

As used herein, the term "similarity value" means a value which indicates the relative similarity or distance between two or more samples, organisms, and/or genotypes. The similarity value can be a direct result from the experiment (for example, DNA-DNA hybridizations) or can be the result of the comparison of two experimental data sets by means of a similarity or distance coefficient, known in the art. Since similarity values and distance values can be easily converted into one another, the distinction between both types is only morphological. Therefore the term "similarity value" is used throughout this document to include both similarity and distance values, except when a distance value is explicitly meant.

As used herein, the term "similarity matrix" means a set of similarity values derived from two or more formats including one-dimensional, two-dimensional, and multi-dimensional arrays, individual experiments of the same data type. The term "matrix", as used herein, does not limit the data to the matrix format; data may be recorded or presented in any format. Since similarity matrices and distance matrices can be easily converted into one another, the distinction between both types is only morphological. Therefore the term "similarity matrix" is used throughout this document to include both similarity and distance matrices, except when a distance matrix are explicitly meant.

In one embodiment of the present invention, the experimental data matrix is used to calculate a similarity or distance between organisms, resulting in a similarity matrix as defined above. A non-limiting example of a similarity matrix is provided in Figure 1-B, wherein the similarity matrix has been calculated from the experimental data matrix shown in Figure 1-A. Similarity matrices may be calculated from experimental data matrices using techniques known in the art.

It is within the scope of the present invention to use a similarity matrix to present classifications between, for example, samples, organisms and/or genotypes. In a non-limiting example, a clustering algorithm might be applied to a similarity matrix to produce a dendrogram or tree. Figure 1-C provides an example of a dendrogram produced from the similarity matrix shown in Figure 1-B.

As used herein, a "composite similarity matrix " refers to a new data matrix resulting from the combination of two or more similarity matrices. A composite similarity matrix may or may not be derived from a single data type. In one non-limiting example, a composite similarity matrix is formed from the combination of two or more similarity matrices derived from the same data type. In another non-limiting example, a composite similarity matrix is formed by combining similarity matrices derived from different experimental data types e.g. a similarity matrix derived from results of a DNA electrophoresis gel of a restriction digest with a similarity matrix derived from quantification of mRNA expression levels.

One aspect of the present invention is a method that can generate a consensus classification from the combination of two or more similarity matrices by constructing a composite similarity matrix.

The invention encompasses methods which may result in a means to view said classifications such as, for example, a dendrogram, a Principal Components Analysis (PCA), a Self-Organizing Map (SOM), a Discriminant Analysis (DA), and other known grouping techniques.

The invention encompasses methods which may result in a means to view phylogenetic evolution, mutational evolution or clonal relationships such as, for example, a phylogenetic tree, an average distance tree, a minimum spanning tree, or any other graphical representation that visualises clonal or evolutionary relationship.

The invention encompasses methods which may evaluate the quality of the resulting dendrogram, grouping or display of evolution by means of a general quality score or quality score at each branching point (co-phenetic correlation, standard deviations, Jackknife and Bootstrap tests) and the invention can use these values to optimise and steer an analysis procedure.

The inventors have found that the use of composite similarity matrices to produce a consensus classification, leads to a surprising increase in the accuracy of the classification so produced. The inventors have further found that the combining of similarity matrices according to a method of the invention, including when diverse experimental datatypes are available, such as, for example similarity matrices derived from DNA electrophoresis experiments and similarity matrices derived from enzymatic or metabolic activity assay studies, the classification so produced is surprisingly more accurate compared with conventional methods of the art.

One embodiment of the present invention is a method suitable for producing a consensus classification of organisms using the data derived from two or more experiments performed on said organisms or samples thereof comprising the steps of:

i) obtaining similarity matrices from the said data,
ii) producing a composite similarity matrix that is a function of said similarity matrices, and
iii) producing a consensus classification from said composite similarity matrix.

Another embodiment of the present invention is a method as defined above wherein the function of step ii) comprises averaging the corresponding elements of said similarity matrices.

Another embodiment of the present invention is a method as defined above wherein each similarity matrix is weighted according the number of experimental characters used to calculate said matrix, to arrive at the average.

Another embodiment of the present invention is a method as defined above wherein each similarity matrix is weighted by a user defined value to arrive at the average.

Another embodiment of the present invention is a method as defined above, wherein said experiments produce product size or retention time results, and wherein the each element of each similarity matrix is weighted according to the number of bands or features associated with that element, to arrive at the average.

Another embodiment of the present invention is a method as defined above wherein said experiments are any of electrophoresis, high performance liquid chromatography, gas chromatography, capillary electrophoresis, chromatography, thin-layer chromatography, and/or mass spectrometry.

Another embodiment of the present invention is a method as defined above wherein the function of step ii) comprises the steps of:

a) linearising said similarity data matrices,

b) averaging the corresponding elements of said linearised similarity matrices of step a)

Another embodiment of the present invention is a method as defined above wherein step a) comprises the minimisation of equations:

$$\sum_{i=1}^{p}\sum_{j=1}^{i-1}\left(\hat{d}_{k,ij}-f_{k}(D_{ij})\right)^{2} , \forall k$$

$$\sum_{i=1}^{p}\sum_{j=1}^{i-1}\left(D_{ij}-g_{k}(\hat{d}_{k,ij})\right)^{2} , \forall k$$

wherein $p$ is the number or organisms, samples or genotypes, wherein each technique k results in a matrix of pairwise distance values, so that the distance value obtained between organism i and j from technique k is given by $d_{k,ij}$, wherein $\hat{d}_{k,ij}=\dfrac{d_{k,ij}}{s_{k}}$ with

$$S_{k} = \frac{2}{(p-1)(p-2)}\sum_{i=1}^{p}\sum_{j=1}^{i-1}d_{k,ij} ,$$

wherein the consensus distance matrix $D_{ij}$ is considered as the unknown true universal distance scale and wherein the goal is to search the consensus distances $D_{ij}$, $g_k$ and $f_k$ so that $d_{k,ij} \cong f_k(D_{ij})$ and $D_{ij} \cong g_k(d_{k,ij})$ hold as true as possible.

Another embodiment of the present invention is an apparatus suitable for performing the methods as defined above.

Another embodiment of the present invention is a computer program comprising a computing routine, stored on a computer readable medium suitable for producing a consensus classification of organisms using the data derived from two or more experiments performed on said organisms or samples thereof comprising according to the methods as defined above.

Another embodiment of the present invention is a device suitable for producing a consensus classification of organisms using the data derived from two or more experiments performed on said organisms or samples thereof comprising according to the methods as defined above.

**Averaging of similarity matrices**

In one embodiment of the present invention, a method of obtaining a consensus classification comprises the steps of:

i) calculating two or more similarity matrices from two or more experimental data matrices, using methods known in the art, wherein the data type of one experimental data matrix may be the same as that of other experimental data matrices in the calculation, or the data type of one experimental data matrix may be different from that of other experimental data matrices in the calculation,

ii) calculating a composite similarity matrix by averaging the corresponding elements of the respective matrices, i.e. between the same pairs of organisms or samples, and

iii) calculating a consensus classification from the composite similarity matrix of step ii).

According to one embodiment of the invention, a composite similarity matrix is calculated by averaging the corresponding elements of the respective similarity matrices. This means, for example, in the case of element 1, that sum of element 1 from similarity matrix 1, element 1 from similarity matrix 2, element 1 from similarity matrix 3 etc. is divided by the number of matrices, so producing an average of element 1 for the similarity matrices used in the calculation.

In one embodiment of the present invention, a method of obtaining a consensus classification comprises the steps of:

i) calculating two or more similarity matrices from two or more experimental data matrices, using methods known in the art, wherein the data type of one experimental data matrix may be the same as that of other experimental data matrices in the calculation, or the data type of one experimental data matrix may be different from that of other experimental data matrices in the calculation,

ii) calculating a composite similarity matrix by averaging the corresponding elements of the respective matrices, i.e. between the same pairs of organisms or samples, wherein each element is weighted by user-defined parameters, and

iii) calculating a consensus classification from the composite similarity matrix of step ii).

In one embodiment of the present invention, a method of obtaining a consensus classification comprises the steps of:

i) calculating two or more similarity matrices from two or more experimental data matrices, using methods known in the art, wherein the data type of one experimental data matrix may be the same as that of other experimental data matrices in the calculation, or the data type of one experimental data matrix may be different from that of other experimental data matrices in the calculation,

ii) calculating a composite similarity matrix by averaging the corresponding elements of the respective matrices, wherein each similarity matrix is weighted according the number of experimental characters used to calculate said matrix, and

iii) calculating a consensus classification from the composite similarity matrix of step ii).

The inventors found that the averaging and weighting method of step ii) in the above embodiments provides a surprising increase in the accuracy of the resulting consensus classification.

According to one embodiment of the present invention, two or more similarity matrices are averaged according to equation [1], wherein Sc is the resulting composite similarity matrix, $S_1$ is similarity matrix 1, $S_2$ and similarity matrix 2, and $S_n$ is similarity matrix n. Equation [1] is an example of combining two or more similarity matrices using unweighted averages.

$$Sc = \frac{S_1 + S_2 + ...... + S_n}{n} \qquad [1]$$

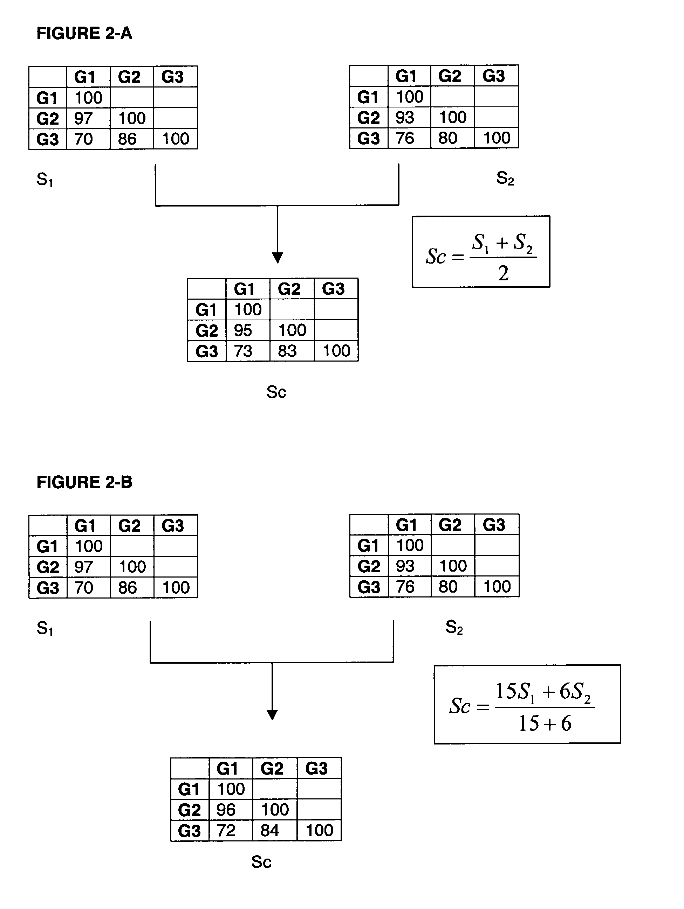

A non-limiting example of the embodiment is provided in Figure 2-A wherein similarity matrix $S_1$ is derived from 15 experimental character types and similarity matrix $S_2$ is derived from 6 experimental character types. The composite similarity matrix so formed is calculated according to equation [1].

According to another embodiment of the present invention, two or more similarity matrices are averaged according to equation [2], wherein Sc is the resulting composite similarity matrix, $S_1$ is similarity matrix 1, a is the number of elements used in the experimental data matrix to calculate similarity matrix 1; $S_2$ is similarity matrix 2, b is the number of elements used in the experimental data matrix to calculate similarity matrix 2; $S_n$ is similarity matrix n, p is the number of elements used in the experimental data matrix to calculate similarity matrix $S_n$. Equation [2] is an example of an equation for combining two or more similarity matrices using weighted averages.

$$Sc = \frac{aS_1 + bS_2 + ...... + pS_n}{(a+b+.....+ p)} \qquad [2]$$

An example of combining similarity matrices using weighted averages based on equation [2], is provided in Figure 2-B, wherein similarity matrix $S_1$ was calculated from an experimental data matrix containing 15 elements and similarity matrix $S_2$ was calculated from an experimental data matrix containing 6 elements. Using weighted averaging, the composite similarity value Sc is calculated according to equation [2].

The averaging method according to equation [2] provides a surprising improvement in the accuracy of the consensus classification so-produced.

In another embodiment of the present invention, the values of a, b...p in Equation [2] are determined by the user. The averaging method wherein the user determines the values of a, b, ...p may also provide an improvement in the accuracy of the so produced consensus classification, when conducted by an experienced user who is familiar with the data types being analysed.

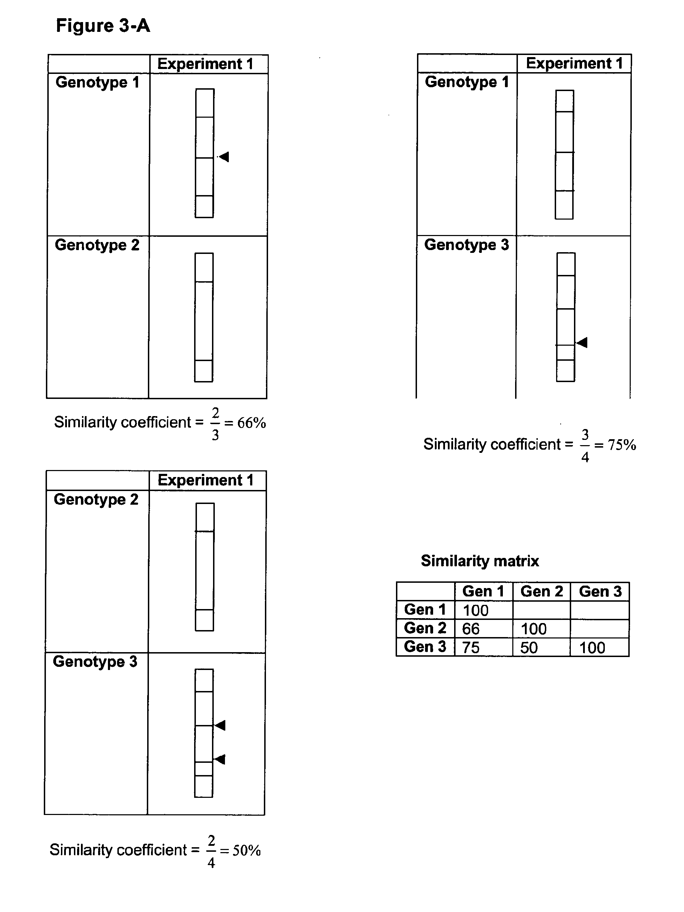

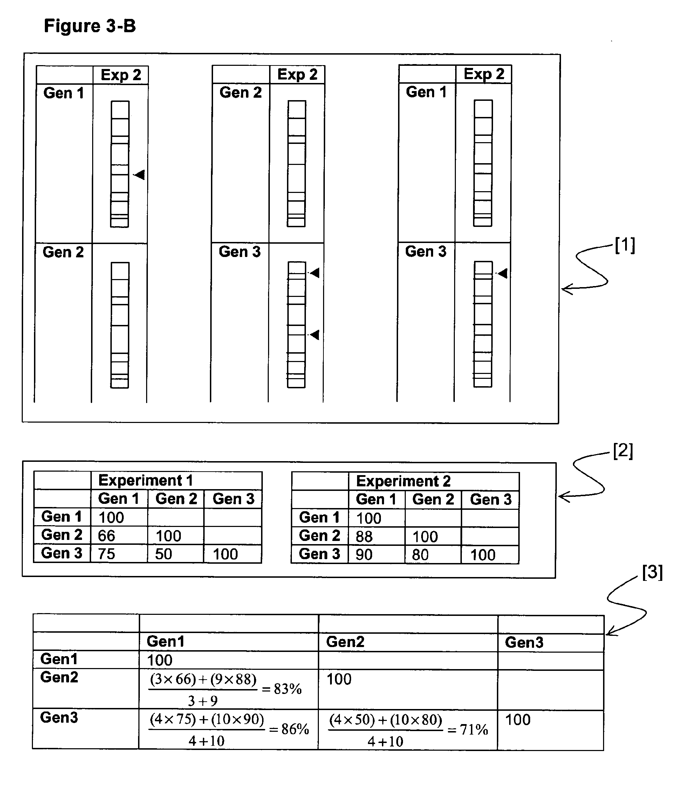

In one embodiment of the present invention, the similarity matrix calculated from experimental data comprising characters belonging to the "product size or retention time" data type, for example DNA electrophoresis gels, is calculated by comparing data sets two by two. Figure 3-A illustrates a non-limiting example wherein the similarity values are calculated from DNA electrophoresis fragment patterns resulting from a restriction digest using a restriction enzyme (experiment 1) and using DNA from three different organisms (genotype 1, 2 and 3). In Figure 3-A, every matching band is considered as one band occurrence, and every unmatched band on either pattern is also a band occurrence. For example in comparing genotype 1 and genotype 2, genotype 1 contains 3 bands , genotype 2 contains 2 bands, 2 bands match, the similarity coefficient would be calculated at 2/3 or 66.667%. Thus the similarity matrix derived from experimental data of the "product size or retention time" data type, is built-up, according to a method of the present invention, by comparing data sets two by two.

In another embodiment of the present invention, similarity matrices derived from experimental data matrices containing characters of the "product size or retention time" data-type may be combined to form a composite similarity matrix by taking the number of bands or features associated with each element of each similarity matrix for use as a weighting constant of that element.

A method of the present invention for combining similarity matrices derived from experimental data matrices containing characters of the "product size or retention time" data type comprises the use of equation [3]. Equation [3] assumes that $N$ product size or retention time data types are combined into a composite similarity matrix, wherein wherein $Sc^e$ is the value of element $e$ of the composite similarity matrix ($Sc$), $S_{D_i}^e$ is the value of element $e$ of the similarity matrix from product size or retention time data type $i$, $p_{D_i}^e$ is the number of experimental features associated with that element, and $S_{D_i}^e$ is the value of element e of the similarity matrix from product size or retention time data type $i$.

$$Sc^e = \frac{\sum_{i=1}^{N} p_{D_i}^e S_{D_i}^e}{\sum_{i=1}^{N} p_{D_i}^e} \qquad\qquad [3]$$

Figure 3-B shows a non-limiting example whereby a composite matrix is calculated from similarity matrices derived from different DNA restriction digest results. Box [1] shows the results of a DNA restriction digest upon the DNA of the organisms of Figure 3-A (Gen 1, 2 and 3), using a different restriction enzyme (exp 2). Box [2] shows the similarity matrix produced therefrom, using the method described for Figure 3-A, and Box [2] also shows the similarity matrix resulting from Exp 1. The elements or similarity coefficients of the composite similarity matrix shown in Box [3] are calculated using equation [3]. For example, the composite similarity value between Gen 1 and Gen 2 (83%) is calculated by weighting the similarity value between Gen 1 and Gen 2 measured in exp 1 (66%) by the number of bands associated with that measurement (3 bands); and weighting the similarity value between Gen 1 and Gen 2 measured in exp 2 (88%) by the number of bands associated with that measurement (9 bands); summing the weighted values (3x66 + 9x88) and dividing by the total number of bands (12 bands) to arrive at the composite similarity value (83%).

The inventors have found that the combining of similarity matrices according to the methods of the invention described above, including when data-types such as those from DNA electrophoresis experiments are available, the consensus classification so produced is surprisingly more accurate compared with conventional methods of the art.

According to another embodiment of the present invention, the averaging of similarity matrices to produce a composite similarity matrix as described above may be generally applied to similarity matrices derived from any combination of experimental data sets or data matrices. It may include the combination of a similarity matrix derived from one data type with a similarity matrix derived from another data type. It may also include the combination of a similarity matrix derived from one data type with a similarity matrix derived from the same data type. It may also include the combination of more than two similarity matrices, each derived from a different data type.

**Linearisation of similarity matrices.**

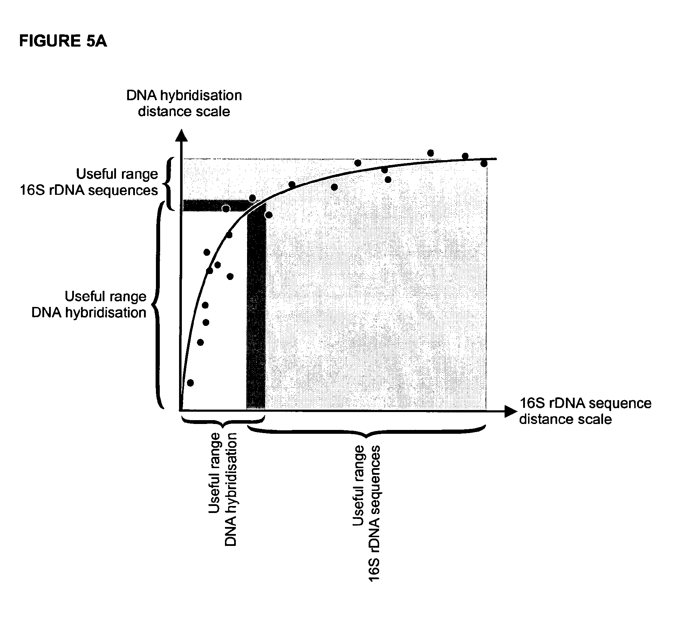

The method described above to create a composite similarity matrix based on the weighted or unweighted *average* of individual similarity or distance matrices works well when the expected discriminatory range for both techniques is comparable, and when the matrices are complete, *i.e.* for each experiment there is a similarity value present for each pair of entries. However, when two experimental techniques are performed on the same set of organisms, and they generate strongly different similarity or distance levels, the composite similarity matrix formed using the method disclosed above can be distorted. For example, DNA hybridisation and16S rDNA sequencing are techniques whose discriminatory ranges are different. Figure 5A compares the distance matrices from DNA hybridisation values and16S rDNA gene sequences. On the scale of 16S rDNA sequence distance (the X-axis), the DNA hybridisation-based distances occupy a narrow range close to zero distance, whereas on the scale of DNA hybridisation (the Y-axis), 16S rDNA sequence distances occupy a narrow range on the most distant side of the scale. This effect is due to the nonlinear relation between both matrices. Both matrices can be considered as zoomed windows on different ranges of a hypothetical linear distance scale. Thus the averaging of similarity matrices derived from experiments with different discrimination ranges can lead to a distorted composite similarity matrix.

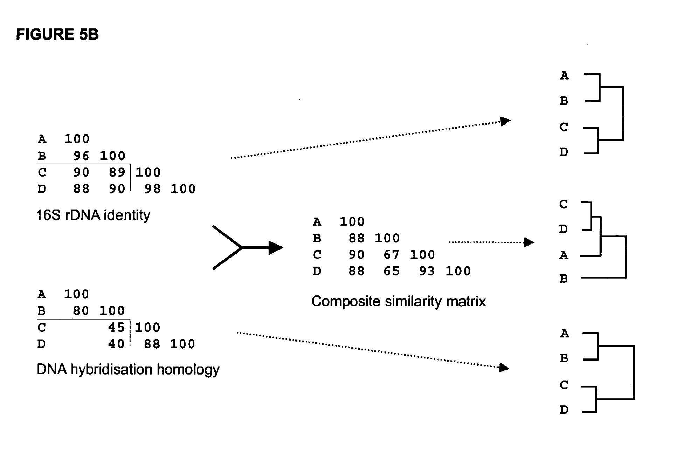

The averaging of two similarity or distance matrices with different ranges wherein one or both matrices contain missing values can even lead to stronger distortion. For example, Figure 5B shows two similarity matrices of values from DNA homology and 16S rDNA sequence experiments on the same organism (A, B, C and D). As shown, DNA homology values range from 88% to 40%, whereas 16S rDNA gene identity ranges between 98% and 88%. In addition, the DNA hybridization homology matrix has some missing elements. In spite of these missing elements, it is clear from the DNA hybridization homology matrix alone that the four genotypes analysed consist of two groups: [A,B] and [C,D]. The 16S rDNA sequence identity matrix also suggests the same groupings, although at a different scale.

The composite similarity matrix created from these two techniques shows averaged values for [AB], [BC], [BD], and [CD] but for [AC] and [AD] it has taken the only available values, 90% and 88%, respectively. The resulting matrix provides a distorted view of the relationships between these three organisms, as it suggests [AC] and [AD] to be at least as closely related as [AB]. The resulting UPGMA dendrogram also depicts a different classification as compared to the two dendrograms derived from DNA hybridisation and 16S rDNA sequence identity individually.

One aspect of the invention is a method for constructing a composite similarity matrix that combines the information present in the individual matrices in a way that the useful information from each of the constituent matrices is optimally preserved, *i.e.* by respecting the particular discriminatory ranges of the techniques applied.

In one embodiment of the present invention, similarity matrices are combined by averaging to form a composite similarity matrix wherein the constituent similarity matrices are linearised prior to averaging.

By "linearise" in reference to a similarity matrix herein is meant adjusting the value of each element of said matrix such that those values that fall in the window considered useful to classification (the range of the technique) are placed on the same linear scale as those useful values of one or more other similarity matrices used in the calculation.

One advantage of using an averaging method incorporating a linearisation step for combining similarity matrices is that the similarity range for both experiments does not need to be comparable.

Another advantage of an averaging method incorporating a linearisation step for combining similarity matrices is that the discriminatory depth (taxonomic depth or phylogenetic depth) of the methods used to construct the constituent similarity matrices do not need to be the same.

Another advantage of using an averaging method incorporating a linearisation step for combining similarity matrices is that the individual similarity matrices do not need to be complete. The method allows incomplete similarity matrices, resulting from experimental data matrices with missing experiments, to be combined successfully without distortion of the composite similarity values towards one of the constituent values.

In one embodiment of the invention, a method for linearising similarity matrices according to the invention comprises a mathematical description to the problem of linearising matrices and a solution therefor. One possible mathematical description and solution therefor for linearising similarity matrices according to the invention is disclosed below.

*Attributes of the problem*

In one mathematical description of the problem according to invention, p organisms, samples or genotypes, and n experimental techniques applied on these organisms, samples or genotypes are considered. Each technique k results in a matrix of pair-wise distance values, so that the distance value obtained between organism *i* and *j* from technique *k* is given *by* $d_{k,ij}$ . In most cases, these distance values are the result of calculating a mathematical distance coefficient on the experimental data sets.

Non-limiting examples are:

- Nucleic acid sequences: number of mutations in aligned sequences
- Banding patterns resulting from electrophoresis of DNA restriction fragments: number of different bands, distance-converted Jaccard or Dice coefficients
- Enzymatic activity profiles: distance-converted product-moment correlation
- Microarrays: Euclidean distance.

According to the invention, experimental data matrices obtained from the techniques are not necessarily complete; some experiments may not have been performed for some organisms or samples, which results in incomplete distance matrices, i.e. distance matrices with missing values. Alternatively, experimental data matrices obtained from the techniques are complete.

*Mathematical description of the problem*

The distance scales from the different experiments are normalized by dividing them by their corresponding Root-Mean-Square (RMS) values according to Equations [4] and [5] below.

$$\hat{d}_{k,ij} = \frac{d_{k,ij}}{S_k} \tag{4}$$

where

$$S_k = \frac{2}{(p-1)(p-2)} \sum_{i=1}^{p} \sum_{j=1}^{i-1} d_{k,ij} \tag{5}$$

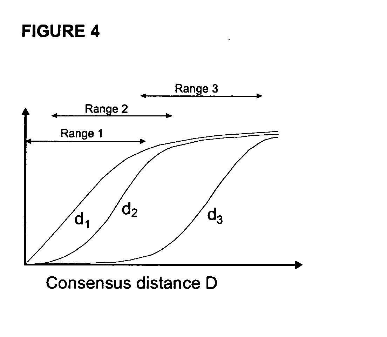

The consensus distance matrix *D* is considered as an unknown "true" universal distance scale, with all the individual distance scales for each experiment being mapped on that universal distance scale by some nonlinear function according to equation [6], wherein each individual experiment *k* has its own functional dependence (Figure 4).

$$\hat{d}_{k,ij} \cong f_k(D_{ij}), \ \forall k,i,j \tag{6}$$

In practical cases, this relationship will not be 100% exact (e.g. due to scatter on the measurements and because of practical limitations of the experiments).

Equivalently, the consensus distances are connected to the individual distances by nonlinear function [7], wherein the ideal case $g_k = f_k^{-1}$.

$$D_{ij} \cong g_k(\hat{d}_{k,ij}), \ \forall k,i,j \tag{7}$$

The goal is to search the functions $f_k$ and $g_k$ so that these relations hold as close as possible. Each function $f_k$ holds information about the range of the experiment *k*.

There are some considerations that put constraints on the functions $f_k$ as follows.

1. Since identical organisms should have zero distance for every possible technique, $f_k(0) = 0$, and consequently $g_k(0) = 0$.
2. On average, the distance $d_{k,ij}$ between a pair of organisms should increase if the "true" biological distance increases. However, due to experimental errors, statistical fluctuation, or imperfections of the technique, this may not be true for particular cases. But, as an overall trend, the inventors have found that it should hold for every well-designed comparison experiment. Hence, all functions $f_k$ should increase monotonically. As a direct consequence, $g_k$ should increase monotonically as well.

Summarising the mathematical description of the problem, the goal is to search the consensus distances $D_{ij}$ and the functions $g_k$ and $f_k$ so that $d_{k,ij} \cong f_k(D_{ij})$ and $D_{ij} \cong g_k(d_{k,ij})$ hold as true as possible. This can be put in a more exact, least square sense, by minimizing equations [8] and [9].

$$\sum_{i=1}^{p} \sum_{j=1}^{i-1} \left( \hat{d}_{k,ij} - f_k(D_{ij}) \right)^2, \ \forall k \tag{8}$$

$$\sum_{i=1}^{p}\sum_{j=1}^{i-1}\left(D_{ij}-g_k(\hat{d}_{k,ij})\right)^2 , \ \forall k$$ [9]

There are many equivalent solutions to this problem. This reflects the fact that only relative differences in distances have a physical interpretation. There is no interpretation of the consensus distance values themselves. For example, applying any monotonically increasing function on the consensus distances will hold a new, equivalent solution.

In the equations, we ignored the fact that some distances $\hat{d}_{k,ij}$ may not be known. However, the extension is everywhere straightforward: the summations should be modified to exclude the absent values. We kept the simple forms in order not to overload the notations and mathematical formulas.

*Mathematical solution to the problem*

In order to further parameterize the problem, one can write the functions $f_k$ and $g_k$ as a linear combination of a number of basis functions according to equations [10] and [11], wherein $m$ is the number of basis functions used, $\boldsymbol{B_l}$ is the basis functions, and $\tilde{f}_{k,l}$ and $\tilde{g}_{k,l}$ are scalar values that are the unknowns of the problem.

$$f_k(d) = \sum_{l=1}^{m} \tilde{f}_{k,l} B_l(d)$$ [10]

$$g_k(d) = \sum_{l=1}^{m} \tilde{g}_{k,l} B_l(d)$$ [11]

One choice for the basis functions (although not the only possibility) are power functions according to equation [12]:

$$B_l(d) = d^l.$$ [12]

These expansions are subject to four constraints:

   1. $f_k(0)=0$,
   2. $g_k(0)=0$,
   3. $f_k$ increases monotonically, and
   4. $g_k$ increases monotonically.

One can account for 1. and 2. by only using basis functions that fulfill the same criterion: $B_l(0) = 0$ . Points 3. and 4. are discussed later below.
The problem is now translated to the minimization of equations [13] and [14], with unknown values $\tilde{f}_{k,l}, \tilde{g}_{k,l}$ and $\boldsymbol{D_{ij}}$.

$$\sum_{i=1}^{p}\sum_{j=1}^{i-1}\left(\hat{d}_{k,ij}-\sum_{l=1}^{m}\tilde{f}_{k,l}B_k(D_{ij})\right)^2 , \ \forall k$$ [13]

$$\sum_{i=1}^{p}\sum_{j=1}^{i-1}\left( D_{ij} - \sum_{l=1}^{m} \widetilde{g}_{k,l} B_k(\hat{d}_{k,ij}) \right)^2, \forall k \qquad [14]$$

Unfortunately, these equations are not linear in the unknowns: equation [13] contains terms that are mixed in $\hat{\imath}_{k,l}$ and $D_{ij}$. Moreover, $B_k (D_{ij})$ may be a nonlinear function. As a consequence, one cannot apply the theory of linear least squares optimization to this problem. One possible solution would be to apply a general nonlinear iterative minimization algorithm (*e.g.* Levenberg-Marquard).

However, in practical cases, it is sufficient to follow a simpler approach, by first minimizing equation [14] for an optimal solution for $\widetilde{g}_{k,l}$ and $D_{ij}$, and then minimizing equation [13] for a solution for $\hat{\imath}_{k,l}$. The inventors have found that this approach yields good results - equation [13] and [14] are to a equivalent to a high degree: they are in fact each other's inverse. A solution that is optimal for one equation will be almost optimal for the other one.

Although equation [14] is linear in the unknown variables, one has to take special precautions in order not to arrive at the trivial, perfect but meaningless solution $D_{ij} = 0$ and $\widetilde{g}_{k,l} = 0$. One solution is to follow an iterative approach as follows:

1. Set the consensus distances equal to the averaged individual distances according to equation [15].

$$D_{ij} = \frac{1}{n}\sum_{k=1}^{n} \hat{d}_{k,ij} \qquad [15]$$

2. Minimize equation [14] for the unknown values $\widetilde{g}_{k,l}$, keeping $D_{ij}$ fixed. This is a standard linear least square problem.
3. Minimize equation [14] for the unknown values $D_{ij}$, keeping $\widetilde{g}_{k,l}$ fixed.
4. Standardize $D_{ij}$ in some way, e.g. by dividing by the total RMS value
5. Return to 2. until convergence is achieved.

Step 2. needs to take into account the fact that $g_k$ must increase monotonically. This can be achieved by using a Quadratic Programming technique rather than a simple least square fit.

Basically, one evaluates the first derivative of $g_k$ in a number of fixed distance values $\bar{d}_i$, $i$=1...$q$, requiring that the first derivative should be non-negative everywhere: $g'_k (\bar{d}_i) \geq 0$, $\forall k,i$. For example, the points $\bar{d}_i$ could be chosen is such a way that they are spread in an equidistant way over the whole distance range. A sufficient number of points needs to be used to ensure a consistent non-negative derivative. In each point $\bar{d}_i$, this condition translates into a linear inequality in the unknowns $_{k,l}$ using equation [16], wherein $B'_l$ the first derivative of the basis function $B_l$.

$$\sum_{l=1}^{m} \widetilde{g}_{k,l} B'_l(\bar{d}_i) \geq 0, \ \forall k,i \qquad [16]$$

The minimization of the quadratic form given by equation [14], together with these linear inequalities are a standard problem that can be solved by methods known in the art, such as Quadratic Programming.

When a solution is obtained for the consensus distances $D_{ij}$, it is still possible to apply any monotonically increasing function $c(d)$ that has $c(0) = 0$. This does not change anything fundamental to the solution and has only a cosmetic interpretation.

**FIGURES**

Figure 1-A, 1-B, 1-C. Example of a conventional cluster analysis based upon 4 organisms each characterized by four characters of the same type.
Figure 2-A, 2-B, Combining similarity matrices using non-weighted and weighted averaging.
Figure 3-A, 3-B. Weighted and unweighted averaging of similarity matrices obtained from DNA electrophoresis banding patterns.

Figure 4. Figure illustrating the differences in active ranges of results of three experiments, d1, d2 and d3.

Fig. 5A. Comparison between distance matrices from two techniques with different discriminatory ranges.

Figure 5B: Example of averaging two similarity matrices with different ranges, and containing missing elements.

Figure 6. *Xba*l and *Avr*ll patterns for six *E. coli* strains belonging to different serotypes, according to Example 1.

Figure 7. UPGMA dendrogram obtained from *Xba*l patterns from six *E. coli* strains, according to Example 1.

Figure 8. UPGMA dendrogram obtained from *Avr*ll patterns from six *E. coli* strains, according to Example 1.

Figure 9. UPGMA dendrogram obtained from unweighted average similarities of *Xba*l and Avrll patterns from six *E. coli* strains.

Figure 10. UPGMA dendrogram obtained from weighted average similarities of *Xba*l and *Avr*ll patterns from six *E. coli* strains.

Figure 11. AFLP clustering of *Xanthomonas.*

Figure 12. DNA hybridization clustering of *Xanthomonas.*

Figure 13. 16S sequence homology clustering of *Xanthomonas.*

Figure 14. Consensus clustering of AFLP, DNA hybridization and 16S rRNA sequences of *Xanthomonas* based upon unweighted average similarity matrix.

Figure 15. Consensus clustering of AFLP, DNA hybridization and 16S rRNA sequences of *Xanthomonas* based upon linearized average similarity matrix.

Figure 16. UPGMA clustering of two known species and one unknown species of genus A.

Figure 17. Histon H3 sequence clustering of different members of a eukaryote genus.

Figure 18. Consensus clustering of fatty acid composition and Histon H3 sequences.

## EXAMPLES

**Example 1:** Pulsed Field Gel Electrophoresis (PFGE) using different restriction enzymes to determine pathotype of *E. coli* O157:H7 strains.

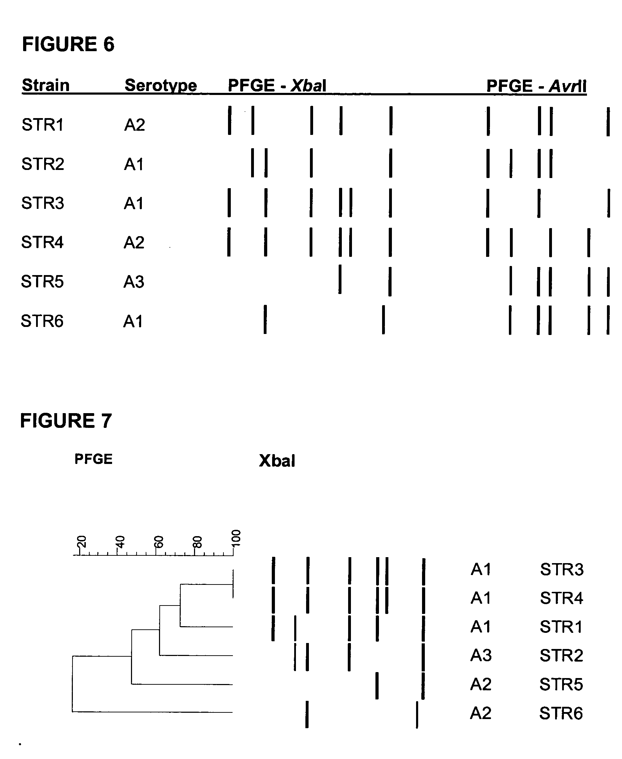

Six pathogenic strains of *E. coli* O157:H7 are analyzed by means of PFGE using two different restriction enzymes: *Xba*l and *Avr*ll. By means of serological tests, the strains have been assigned to specific serotypes. The results of the experiments are shown in Figure 6

The similarities between the strains are calculated using the Dice coefficient:

$$\frac{2N_{[AB]}}{N_A + N_B}$$

where $N_A$ is the total number of bands in pattern A, $N_B$ the total number of bands in pattern B, and $N_{[AB]}$ the number of common bands between patterns A and B.

A. PFGE-*Xba*l

Using the Dice coefficient on the experimental data for PFGE-*Xba*l depicted in Figure 1, produces the similarity matrix in Table 1.

Table 1.

| Similarity matrix of *Xba*l patterns obtained from six *E. coli* strains. | | | | | |
|---|---|---|---|---|---|
| **STR1** | 100.00 | | | | |
| **STR2** | 66.67 | 100.00 | | | |
| **STR3** | 72.73 | 60.01 | 100.00 | | |
| **STR4** | 72.73 | 60.01 | 100.00 | 100.00 | |
| **STR5** | 57.15 | 33.33 | 50.00 | 50.00 | 100.00 |
| **STR6** | 0.00 | 33.33 | 25.00 | 25.00 | **0.00** | 100.00 |

Cluster analysis using the unweighted pair group method with arithmetic averages (UPGMA) results in the dendrogram shown in Figure 7. PFGE-*Xba*l patterns are able to distinguish A1 serotype strains from the others, as they cluster together. A2 serotype strains, however, could not be clustered together as shown by the dendrogram in Figure 7. Furthermore, no distinction is possible between strains 3 and 4 from serotype A1.

B. PFGE-*Avr*II

Using the Dice coefficient on the experimental data for PFGE-*Avr*II depicted in Figure 6, produces the similarity matrix in Table 2.

Table 2.

| Similarity matrix of *Avr*II patterns obtained from six *E. coli* strains. | | | | | | |
|------|--------|--------|--------|--------|--------|--------|
| **STR1** | 100.00 | | | | | |
| **STR2** | 75.01 | 100.00 | | | | |
| **STR3** | 85.72 | 57.15 | 100.00 | | | |
| **STR4** | 50.00 | 75.01 | 28.57 | 100.00 | | |
| **STR5** | 66.67 | 66.67 | 50.00 | 66.67 | 100.00 | |
| **STR6** | 66.67 | 66.67 | 50.00 | 66.67 | **100.00** | 100.00 |

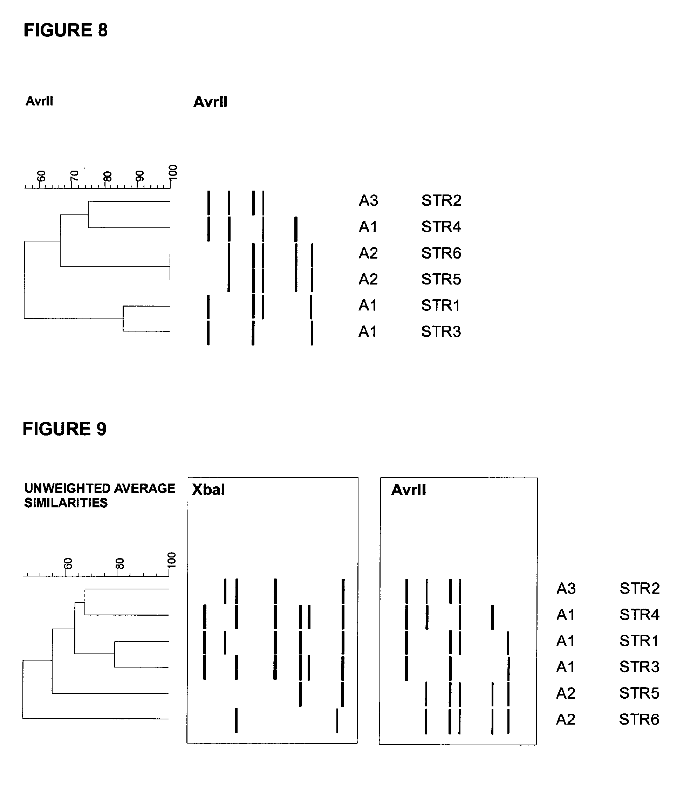

Cluster analysis using the unweighted pair group method with arithmetic averages (UPGMA) results in the dendrogram shown in Figure 8. PFGE-*Avr*II patterns are able to distinguish A2 serotype strains from the others, as they cluster together according to the dendrogram in Figure 8. A1 serotype strains, however, could not be clustered together. Furthermore, no distinction is possible between the two strains from serotype A2.

C. Combined clustering of *Xba*I - *Avr*II by unweighted averaging

In a third analysis, the two similarity matrices (*Xba*I and *Avr*II) are combined to create a new consensus matrix by averaging the corresponding values of the two matrices using unweighted arithmetic averages. This results in the following composite matrix (Table 3):

Table 3.

| Composite similarity matrix obtained by unweighted averaging of *Xba*I and *Avr*II similarities from six *E. coli* strains. | | | | | | |
|------|--------|--------|--------|--------|--------|--------|
| **STR1** | 100.00 | | | | | |
| **STR2** | 70.84 | 100.00 | | | | |
| **STR3** | 79.23 | 58.58 | 100.00 | | | |
| **STR4** | 61.37 | 67.51 | 64.29 | 100.00 | | |
| **STR5** | 61.91 | 50.00 | 50.00 | 58.33 | 100.00 | |
| **STR6** | 33.33 | 50.00 | 37.50 | 45.83 | **50.00** | 100.00 |

When a UPGMA dendrogram is calculated from this composite matrix, the consensus clustering obtained is shown in Figure 9. The linear combination of both techniques is able to distinguish every strain from every other. However, serotype A1 strains, and in particular serotype A2 strains are still not grouped together. When looking more closely at the similarities between the A2 group strains STR5 and STR6, we find the two bands to be different in *Xba*I analysis, whereas all 5 bands are the same in *Avr*II analysis. This results in 0% and 100% similarity in the respective matrices (see above). The unweighted average matrix consequently shows 50% similarity between the two strains, and the UPGMA algorithm does not cluster them together.

D. Combined clustering of *Xba*I - *Avr*II by weighted averaging

In a fourth analysis, the two similarity matrices *(Xba*I and *Avr*II) are combined to create a new compopsite matrix by averaging the corresponding values of the two matrices using weighted arithmetic averages. The weights are determined thus that they compensate for the amount of information produced by each pair of experiments. The formula used for the consensus similarity $S_c$ is given the formula:

$$S_c = \frac{N_A S_A + N_B S_B}{N_A + N_B}$$

[4]

$N_A$, $N_B$ being the number of characters in experiments A and B, respectively, and $S_A$ and $S_B$ being the similarity in experiments A and B, respectively. Taking weighted averages results in the composite matrix shown in Table 4:

Table 4.

| Composite similarity matrix obtained by weighted averaging of *Xba*I and *Avr*II similarities from six *E. coli* strains. | | | | | | |
|---|---|---|---|---|---|---|
| STR1 | 100.00 | | | | | |
| STR2 | 70.59 | 100.00 | | | | |
| STR3 | 77.79 | 58.83 | 100.00 | | | |
| STR4 | 63.17 | 66.67 | 73.69 | 100.00 | | |
| STR5 | 62.51 | 53.33 | 50.00 | 58.83 | 100.00 | |
| STR6 | 37.50 | 53.33 | 37.50 | 47.06 | **71.43** | 100.00 |

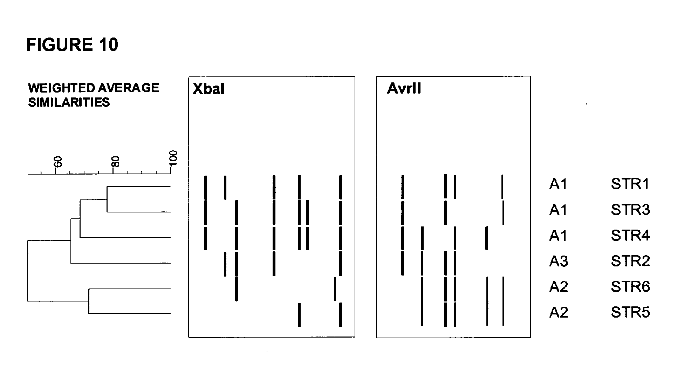

When a UPGMA dendrogram is calculated from this composite matrix, the consensus clustering obtained is that shown in Figure 10. Again, every strain is separated from every other. In addition, the strains are grouped according to serotype. When looking at the serotype A2 strains STR5 and STR6, they share a similarity of 71.43%, which is the weighted average based upon 2 bands in *Xba*I and 5 bands in *Avr*II:

$$\frac{(2 \times 0\%) + (5 \times 100\%)}{2 + 5} = 71.43\%$$

PFGE with *Xba*I only produces two bands per strain, which is far below the minimum to obtain a reasonably significant measure of similarity. Five bands as produced by *Avr*II is still a low number, although more reliable than two. Provided that the experiments are merged with equal weight per observation (as obtained in formula [4]), a new composite experiment is created containing 7 observations, which results in the more reliable clustering as obtained.

**Example 2:** Classification of the genus *Xanthomonas* based upon 3 techniques: AFLP, DNA hybridization, and 16S rRNA gene sequencing.

A total number of 29 strains belonging to the genus *Xanthomonas* together with the type strain of its closest neighbor genus, *Stenotrophomonas,* have been analysed using three genomic techniques: (i) AFLP (Amplified Fragment Length Polymorphism), an electrophoresis technique in which some 30-80 bands are selectively amplified and electrophorized after total genomic restriction analysis; (ii) DNA hybridization, a technique in which the renaturation rate of a mixture of equimolar amounts of total genomic DNA from two organism is measured, to determine the overall homology between the genomes; (iii) 16S rRNA sequence analysis, in which a complete or a partial sequence of the 16S ribosomal RNA gene is determined, and sequences from different organisms compared by homology.

### A. AFLP

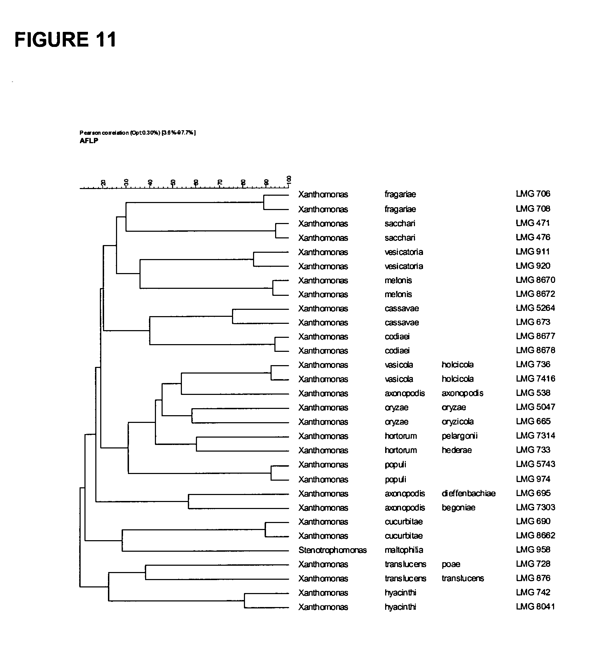

In *Xanthomonas,* as in many other bacterial genera, AFLP is able to distinguish between individual strains, and is suitable for strain typing such as done in epidemiological and strain authentication studies. Thanks to the large number of bands revealed by the technique, the range of discrimination extends to the variety, subspecies or even species level. In the present example of *Xanthomonas,* strains from the same species are usually grouped together (except the more heterogeneous species *X. axonopodis),* whereas within the species, the technique is able to distinguish between pathovars (see Figure 11). This is reflected in the cases where strains that belong to the same species but constitute different pathovars (*X. oryzae, X. axonopodis, X. hortorum, X. translucens*): these strains have consequently lower similarities with each other than strains that are not classified in different pathovars (*X. fragariae, X. saccharri, X. vesicatoria, X. melonis, X. cassavae, X. codiaei, X. vasicola* pv. *holcicola, X. populi, X. cucurbitae,* and *X. hyacinthi*).

However, deeper phylogenies such as the relationships between the species, and in particular, the relationship between its adjacent genus *Stenotrophomonas,* are not reflected, and the technique would falsely suggest that *Stenotrophomonas maltophilia* is a member of the genus *Xanthomonas.*

The obtained similarity range in the AFLP study is between 10.3% and 96.3%.

B. DNA hybridization

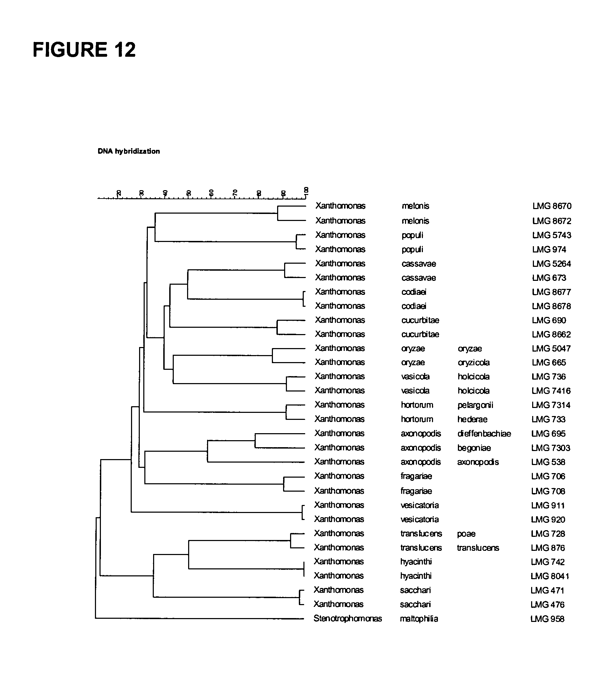

DNA hybridization is a technique which typically differentiates at the species level. Different species can be distinguished from each other by low renaturation rates whereas within the same species, renaturation rates are usually high. Figure 12 shows a clustering of DNA renaturation rates between the same strains as used in the AFLP comparison. Unlike AFLP, the technique is not well suited to distinguish pathovars from one another: most within-species linkage levels are below the experimental error of the technique, which is known to be about 6%. Deeper phylogenies, however, are better revealed than by AFLP. This is reflected by the fact that all species, including *X. axonopodis,* can be separated from each other. Furthermore, a deeper group is formed by the species *X. translucens, X. hyacinthi,* and *X. sacchari,* whereas *Stenotrophomonas maltophilia* is the most distantly clustered strain.

The similarity range of the present DNA hybridization study is between 11.3 and 100%.

C. 16S rRNA sequence comparison

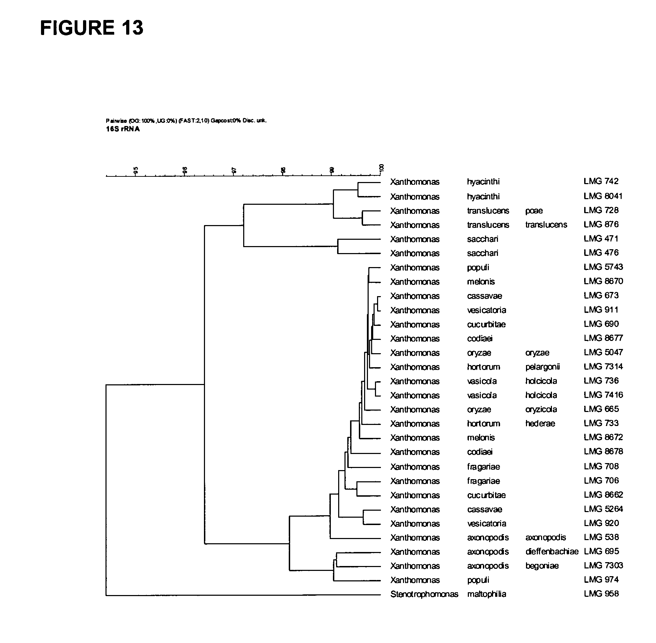

As opposed to most other classification techniques, 16S rRNA sequencing is known to reveal deep phylogenetic relationships (genus and below). Figure 13 displays a cluster analysis of aligned 16S rRNA sequences from the same strains as described before.The dendrogram reveals a strongly different taxonomic structure as compared to AFLP and DNA hybridization. Within *Xanthomonas,* three phylogenetic groups can be found, represented by a main core consisting of most species (A), a separate group formed by the species *X. hyacinthi* and *X. translucens,* affecting monocotyls (B), and a third group formed by *X. sacchari* (from sugarcane) (C). The latter two groups were also suggested by DNA hybridization (Figure 12), but are much more prevalent in the 16S rRNA sequence clustering. Distantly separated from *Xanthomonas* is the *Stenotrophomonas maltophilia* type strain. On the other hand, within-and between-species relationships are not dissolved by 16S rRNA sequencing.
The similarity range of the present 16S rRNA sequence analysis study is between 94.4% and 99.9%.

It should be stressed that there is no conflict between the clustering revealed by AFLP and DNA hybridization on the one hand, and 16S rRNA sequences on the other hand. What actually happens is that each technique has its own window in the phylogenetic space, and grouping analyses such as dendrograms should be looked at within that space. In a technique such as AFLP, grouping levels below 40-50% should not be looked at, because at lower similarity levels, the number of incidentally matching bands becomes statistically too important compared to the number of real matching bands, i.e., which are identical. Likewise, DNA hybridization does not provide reliable similarity values below 40% because of the high experimental error of the technique with too different genomic DNA samples. On the other hand, 16S rRNA sequences do not allow taxonomic distances to be calculated from one or a few different bases out of 1500. Only when a significant number of bases are different (*e.g.* 10 or more), the calculated distances become statistically significant.

The obvious problem in studies like this is how to obtain one consensus clustering, thereby respecting the taxonomic level of each technique, and thus preserving all the information offered by the techniques used. When the similarities of the constitutive matrices are averaged to obtain an average similarity matrix, the small but very notable differences obtained by 16S rRNA sequencing are mostly masked by the large similarity fluctuations obtained by DNA hybridization and, in particular, AFLP. Indeed, the 16S rRNA sequence similarity ranges between 94.7% and 99.9%, meaning a total span of less than 6% between the most distant strains. This is much less than the span of the other techniques (approximately 90%), and is even less than the experimental error of DNA hybridization.

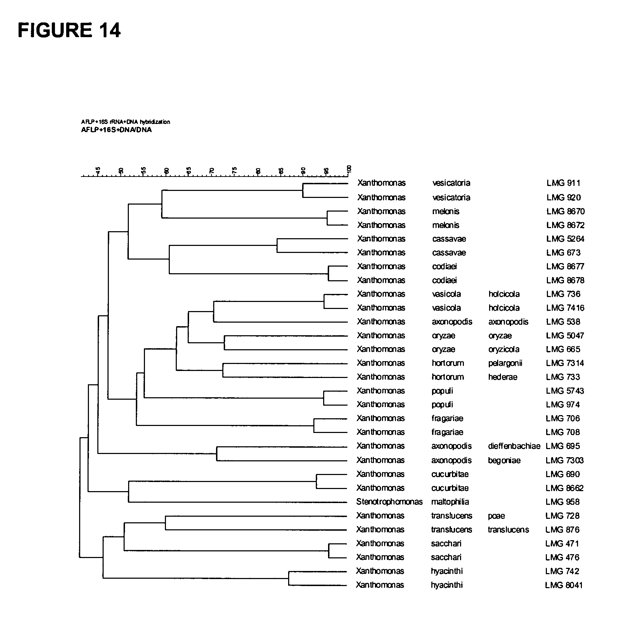

The logical result is a tree that reflects the species and pathovar classification, but does not well reflect the deeper phylogenetic structure of the genus as revealed by 16S rRNA sequences (Figure 14).

An alternative method, using weighted averages, is not applicable to the combination of these techniques, since AFLP patterns in the present study are compared as densitometric curves of 2000 values by means of the Pearson product-moment correlation coefficient, whereas 16S rRNA sequences are composed of 1500 bases on average. These two measures are clearly uncomparable in terms of deriving weighted averages. In addition, DNA hybridization values are undefined in terms of numbers of characters.

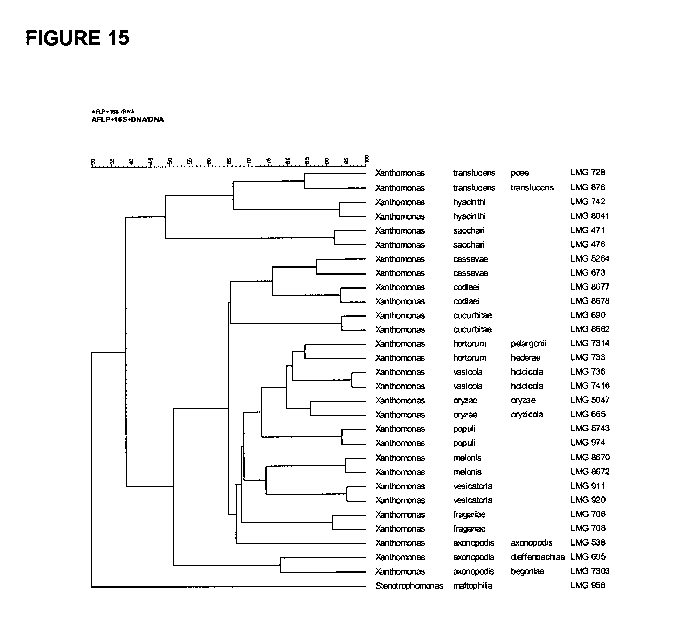

A method according to the invention of linearising similarity matrices which have been derived from different experimental techniques *i.e.* different data types, has been applied to the current data set. The composite similarity matrix resulting from combining said linearised similarity matrices according to the invention produces the dendrogram shown in Figure 15.

The method successfully combines the information delivered by the described techniques into a consensus clustering which reflects both the deeper phylogenetic relationships of the genera and the species, pathovar, and even strain subdivision of the genus *Xanthomonas.* This study shows that the invention presents an invaluable tool for investigating the taxonomic and phylogenetic structure of bacterial taxa, and by extension, of all living organisms that are analyzed by a combination of techniques that reveal information at different taxonomic depth. Consequently, the method can also be of great use for identification purposes, by placing unknown organisms into known classification schemes thereby respecting the level of information offered by each technique used.

**Example 3:** Grouping members of a eukaryote genus based on fatty acid cell wall composition analysis using HPLC and Histone H3 sequencing.

A total number of 15 individuals belonging to a eukaryote genus (taxon not further specified) are analyzed by their phenotype using HPLC analysis of cell wall fatty acid composition, and by genotype using histon H3 sequencing. The members are sampled from different sources: neutral, alkaline, and acid.

A. Fatty acid cell wall composition

HPLC based Fatty Acid Methyl Ester (FAME) analysis is a very sensitive technique which usually allows individual organisms to be separated from one another. The obtained fatty acid metyl ester profiles are very sensitive to environmental influences such as substrate, acidity, temperature. The fatty acid profiles are shown as percentages of total fatty acid amount in Table 5.

| Sample# | Organism | Source | 14:0 | 15:0 | 16:0 | 15:1 ω6c | SumIn #7 | SUM #7 | 10:0 3OH | 17:0 |
|---|---|---|---|---|---|---|---|---|---|---|
| 52441 | A.sylvestris | Neutral | 4.95 | 0 | 22.57 | 0 | 22.36 | 22.36 | 0 | 0 |
| 52417 | A. sylvestris | Neutral | 4.55 | 0 | 25.97 | 0 | 18.5 | 18.5 | 0 | 0 |
| 52418 | A. sylvestris | Neutral | 4.87 | 0 | 25.13 | 0 | 26.23 | 26.23 | 0 | 0 |
| 52435 | A. sylvestris | Neutral | 4.9 | 0 | 22.11 | 0 | 24.08 | 24.08 | 0 | 0 |
| 52414 | A. sylvestris | Alkaline | 3.71 | 0 | 17.55 | 0 | 27.17 | 27.17 | 0.68 | 0 |
| 52434 | A. sylvestris | Alkaline | 3.77 | 0 | 20.53 | 0 | 29.19 | 29.19 | 0.61 | 0 |
| 52416 | A. sylvestris | Acid | 4.05 | 0.24 | 23.43 | 0.25 | 35.27 | 35.27 | 0 | 0.39 |
| 52412 | A. sylvestris | Acid | 4.05 | 0 | 18.24 | 0 | 34.68 | 34.68 | 0 | 0 |
| 52449 | A. aberrans | Neutral | 9.33 | 5.84 | 21.1 | 0 | 12.97 | 12.97 | 0 | 2.01 |
| 52442 | A. aberrans | Neutral | 11.33 | 1.19 | 25.65 | 0 | 11.62 | 11.62 | 0 | 0.53 |
| 52415 | A. sp. | Neutral | 3.26 | 0.9 | 26.16 | 0 | 17.32 | 17.32 | 0 | 0.78 |
| 52424 | A. sp. | Neutral | 2.61 | 0.81 | 28.31 | 0 | 18.58 | 18.58 | 0 | 0.69 |
| 52440 | A. sp. | Acid | 4.16 | 2.03 | 27.68 | 0 | 20.4 | 20.4 | 0 | 1.06 |
| 52452 | A. aberrans | Acid | 3.32 | 0 | 17.02 | 0 | 19.28 | 19.28 | 1.5 | 0 |
| 52430 | A. aberrans | Acid | 3.93 | 0 | 17.69 | 0 | 19.15 | 19.15 | 0.73 | 0 |

Table 5. HPLC Fatty acid profiles obtained from 15 members of a eukaryote genus

| Sample# | 16:1 w5c | SumIn#3 | SumIn#4 | 17:0 Cyclo | 16:1 2OH | 16:0 2OH | 16:0 3OH | 18:0 | 19:0 Cyclo ω8c | 18:1 2OH | SUM#3 |
|---|---|---|---|---|---|---|---|---|---|---|---|
| 52441 | 0 | 7.01 | 7.39 | 11.85 | 1.77 | 1.49 | 6.89 | 0.72 | 5.23 | 6.88 | 7.26 |
| 52417 | 0 | 6.58 | 11.36 | 13.27 | 1.4 | 1.49 | 6.21 | 1.08 | 7.2 | 2.02 | 6.58 |
| 52418 | 0 | 7.36 | 11.81 | 8.58 | 1.12 | 1 | 6.19 | 0.58 | 3.95 | 3.19 | 7.36 |
| 52435 | 0 | 7.49 | 13.78 | 9.17 | 2.55 | 2.2 | 6.1 | 0.78 | 3.97 | 2.56 | 7.49 |
| 52414 | 0 | 15.15 | 6.39 | 7.62 | 1.55 | 1.49 | 5.53 | 0.79 | 4.59 | 4.77 | 15.15 |
| 52434 | 0 | 11.85 | 8.37 | 7.27 | 0.97 | 0.97 | 4.29 | 0.78 | 3.81 | 3.68 | 12.43 |
| 52416 | 0.17 | 5.39 | 17.01 | 2.8 | 1.02 | 0.9 | 4.5 | 0.64 | 2.11 | 1.5 | 5.54 |
| 52412 | 0 | 6.68 | 11.67 | 4.93 | 1.93 | 1.89 | 6.01 | 0.7 | 3.12 | 6.1 | 6.68 |
| 52449 | 0 | 14.64 | 9.47 | 15 | 0 | 0 | 0 | 0 | 1.14 | 0 | 15.08 |
| 52442 | 0 | 14.97 | 7.24 | 15.46 | 0 | 0 | 0 | 0 | 4.83 | 0 | 15.3 |
| 52415 | 0 | 16.24 | 11.82 | 9.55 | 0 | 0 | 0 | 0 | 0.96 | 0 | 16.55 |
| 52424 | 0 | 13.71 | 11.07 | 10.54 | 0 | 0 | 0 | 0.27 | 1.26 | 0 | 14.06 |
| 52440 | 0 | 10.07 | 13.14 | 11.83 | 0 | 0 | 0 | 0.35 | 1.2 | 0 | 10.32 |
| 52452 | 0 | 19.37 | 4.82 | 8.67 | 0.89 | 1.06 | 4.02 | 0.78 | 8.03 | 3.33 | 20.53 |
| 52430 | 0 | 17.27 | 3.5 | 12.4 | 1.53 | 1.49 | 5.01 | 0.57 | 7.99 | 4.2 | 18.04 |

Table 5 (continued)

| Sample# | SUM#4 | 12:0 | 13:0 | 14:0 2OH | 19:0 ISO | 12:0 2OH | SumIn#2 | 15:0 3OH | 13:1 AT 12-13 |
|---|---|---|---|---|---|---|---|---|---|
| 52441 | 7.39 | 0 | 0 | 0 | 0 | 0 | 0 | 0 | 0.66 |
| 52417 | 11.36 | 0 | 0 | 0 | 0 | 0 | 0 | 0 | 0.38 |
| 52418 | 11.81 | 0 | 0 | 0 | 0 | 0 | 0 | 0 | 0 |
| 52435 | 13.78 | 0 | 0 | 0 | 0 | 0 | 0 | 0 | 0.32 |
| 52414 | 6.39 | 0 | 0 | 0 | 0 | 0 | 0 | 0 | 0 |
| 52434 | 8.37 | 0 | 0 | 0 | 0 | 0 | 0 | 0 | 0.44 |
| 52416 | 17.01 | 0 | 0 | 0 | 0 | 0 | 0 | 0 | 0.19 |
| 52412 | 11.67 | 0 | 0 | 0 | 0 | 0 | 0 | 0 | 0 |
| 52449 | 9.47 | 5.17 | 1.07 | 0 | 0 | 0 | 1.52 | 0.29 | 0 |
| 52442 | 7.24 | 3.01 | 0 | 1.77 | 0 | 0.97 | 0 | 0 | 0 |
| 52415 | 11.82 | 6.4 | 0.24 | 3.89 | 0 | 0 | 0 | 0 | 0 |
| 52424 | 11.07 | 5.71 | 0.27 | 3.83 | 0 | 0 | 0 | 0 | 0 |
| 52440 | 13.14 | 4.44 | 0.25 | 2.18 | 0 | 0 | 0 | 0 | 0 |
| 52452 | 4.82 | 0 | 0 | 0 | 0 | 0 | 0 | 0 | 0.5 |
| 52430 | 3.5 | 0 | 0 | 0 | 0 | 0 | 0 | 0 | 0.56 |

Table 5 (continued)

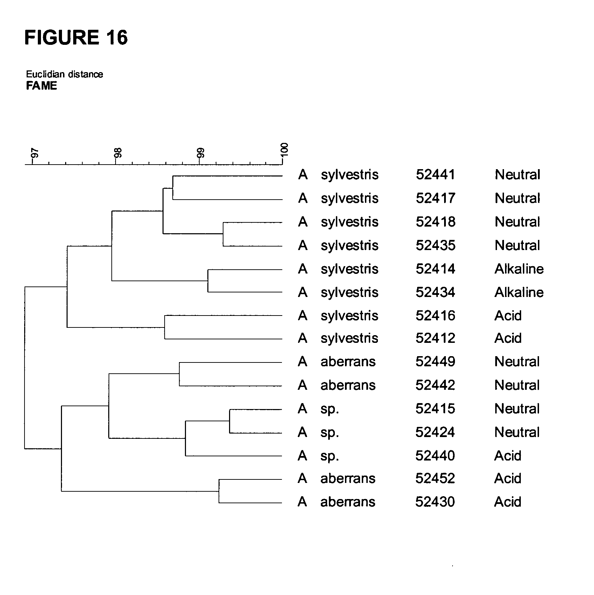

When a UPGMA dendrogram is generated based on Euclidean distance calculation between the fatty acid profiles, the grouping obtained is as shown in Figure 16. Members of the same species from different sources (neutral, alkaline and acid) are well separated from each other, which suggests that the technique allows the origin of a sample to be identified based upon its fatty acid profile. Between the species, however, the separation is not very clear, especially since A. sp. and *A. aberrans* do not occur in separate clusters.

## B. HISTON H3 SEQUENCE CLUSTERING

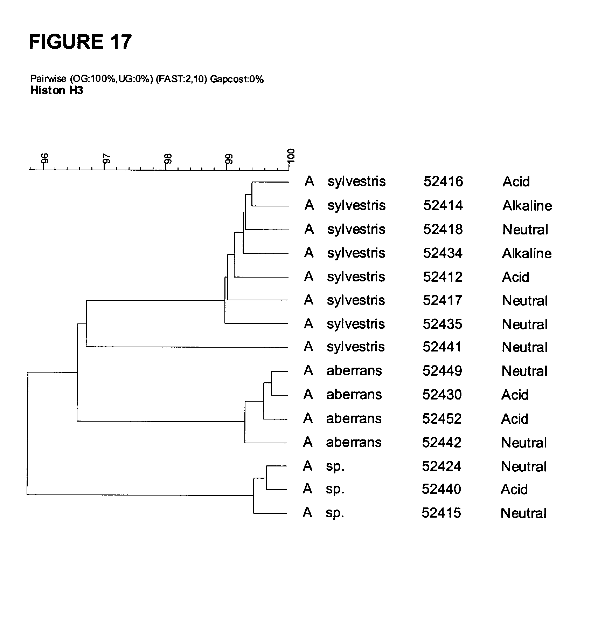

Histon H3 sequences of approximately 520 base pairs long were aligned and clustered to reveal the deeper relationships between the species. The result is a dendrogram depicted in Figure 17.

The separation of the two known species and the unknown species is very pronounced, and apparently, within *A. sylvestris,* there is a phylogenetically aberrant member, which could not be discovered as such using fatty acid composition analysis. On the other hand, it is not possible, using Histon H3 sequence analysis, to discriminate between members sampled from sources with different pH. This is not surprising, as in the short term, environmental factors will influence the phenotype of the organisms rather than the genotype.

## C. CONSENSUS CLASSIFICATION OF FATTY ACID COMPOSITION AND HISTON H3 SEQUENCES

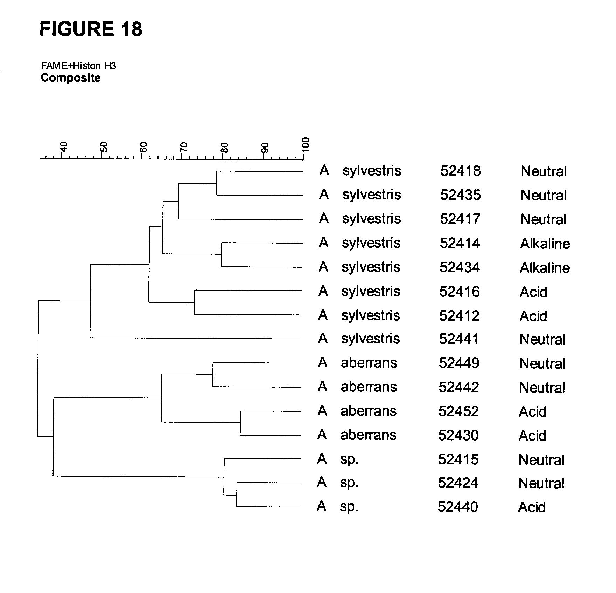

When a consensus matrix is calculated from the individual similarity matrices calculated from fatty acid composition and Histon H3 sequences, the dendrogram obtained is as shown in Figure 18.

The species subdivision as suggested by the Histon H3 sequences is preserved in the clustering, while the phenotypic information as obtained from fatty acid composition analysis is also reflected in the tree. Interestingly, the phylogenetically aberrant *A. sylvestris* member 52441 is not clustered along with the other *A. sylvestris* members from neutral source, but is placed separately based on its sequence divergence.

**Claims**

1. A method suitable for producing a consensus classification of organisms using the data derived from two or more experiments performed on said organisms or samples thereof comprising the steps of:

    i) obtaining similarity matrices from the said data,
    ii) producing a composite similarity matrix that is a function of said similarity matrices, and
    iii) producing a consensus classification from said composite similarity matrix.

2. A method according to claim 1 wherein the function of step ii) comprises averaging the corresponding elements of said similarity matrices.

3. A method according to claim 2 wherein each similarity matrix is weighted according the number of experimental characters used to calculate said matrix, to arrive at the average.

4. A method according to claim 2 wherein each similarity matrix is weighted by a user defined value to arrive at the average.

5. A method according to claim 2, wherein said experiments produce product size or retention time results, and wherein the each element of each similarity matrix is weighted according to the number of bands or features associated with that element, to arrive at the average.

6. A method according to claim 5 wherein said experiments are any of electrophoresis, high performance liquid chromatography, gas chromatography, capillary electrophoresis, chromatography, thin-layer chromatography, and/or mass spectrometry.

7. A method according to claim 1 wherein the function of step ii) comprises the steps of:

    a) linearising said similarity data matrices,
    b) averaging the corresponding elements of said linearised similarity matrices of step a)

8. A method according to claim 7 wherein step a) comprises the minimisation of equations:

$$\sum_{i=1}^{p}\sum_{j=1}^{i-1}\left(\hat{d}_{k,ij} - f_k(D_{ij})\right)^2 \; , \; \forall k$$

$$\sum_{i=1}^{p}\sum_{j=1}^{i-1}\left(D_{ij} - g_k(\hat{d}_{k,ij})\right)^2 , \ \forall k$$

wherein $p$ is the number or organisms, samples or genotypes, wherein each technique k results in a matrix of pairwise distance values, so that the distance value obtained between organism i and j from technique k is given by $d_{k,ij}$, wherein $\hat{d}_{k,ij} = \dfrac{d_{k,ij}}{S_k}$ with

$$S_k = \frac{2}{(p-1)(p-2)}\sum_{i=1}^{p}\sum_{j=1}^{i-1} d_{k,ij} ,$$

wherein the consensus distance matrix $D_{ij}$ is considered as the unknown true universal distance scale and wherein the goal is to search the consensus distances $D_{ij}$, $g_k$ and $f_k$ so that $d_{k,ij} \cong f_k(D_{ij})$ and $D_{ij} \cong g_k(d_{k,ij})$ hold as true as possible.

9. An apparatus suitable for performing the methods according to claims 1 to 8.

10. A computer program comprising a computing routine, stored on a computer readable medium suitable for producing a consensus classification of organisms using the data derived from two or more experiments performed on said organisms or samples thereof according to the methods of claims 1 to 8.

11. A device suitable for producing a consensus classification of organisms using the data derived from two or more experiments performed on said organisms or samples thereof according to the methods of claims 1 to 8.

**Figure 1-A**

| | | Experiment Number | | | |
|---|---|---|---|---|---|
| | | Experiment 1 | Experiment 2 | Experiment 3 | Experiment 4 |
| Sample Number | Genotype 1 | 13 | 27 | 5 | 52 |
| | Genotype 2 | 38 | 54 | 28 | 50 |
| | Genotype 3 | 18 | 36 | 17 | 60 |
| | Genotype 4 | 17 | 25 | 7 | 50 |

**Figure 1-B**

| | G1 | G2 | G3 | G4 |
|---|---|---|---|---|
| G1 | 100 | | | |
| G2 | 68 | 100 | | |
| G3 | 77 | 96 | 100 | |
| G4 | 97 | 85 | 72 | 100 |

**Figure 1-C**

**FIGURE 2-A**

|     | G1  | G2  | G3  |
| --- | --- | --- | --- |
| G1  | 100 |     |     |
| G2  | 97  | 100 |     |
| G3  | 70  | 86  | 100 |

S₁

|     | G1  | G2  | G3  |
| --- | --- | --- | --- |
| G1  | 100 |     |     |
| G2  | 93  | 100 |     |
| G3  | 76  | 80  | 100 |

S₂

$$Sc = \frac{S_1 + S_2}{2}$$

|     | G1  | G2  | G3  |
| --- | --- | --- | --- |
| G1  | 100 |     |     |
| G2  | 95  | 100 |     |
| G3  | 73  | 83  | 100 |

Sc

**FIGURE 2-B**

|     | G1  | G2  | G3  |
| --- | --- | --- | --- |
| G1  | 100 |     |     |
| G2  | 97  | 100 |     |
| G3  | 70  | 86  | 100 |

S₁

|     | G1  | G2  | G3  |
| --- | --- | --- | --- |
| G1  | 100 |     |     |
| G2  | 93  | 100 |     |
| G3  | 76  | 80  | 100 |

S₂

$$Sc = \frac{15S_1 + 6S_2}{15 + 6}$$

|     | G1  | G2  | G3  |
| --- | --- | --- | --- |
| G1  | 100 |     |     |
| G2  | 96  | 100 |     |
| G3  | 72  | 84  | 100 |

Sc

# Figure 3-A

| | Experiment 1 |
|---|---|
| **Genotype 1** | |
| **Genotype 2** | |

Similarity coefficient = $\dfrac{2}{3}$ = 66%

| | Experiment 1 |
|---|---|
| **Genotype 1** | |
| **Genotype 3** | |

Similarity coefficient = $\dfrac{3}{4}$ = 75%

| | Experiment 1 |
|---|---|
| **Genotype 2** | |
| **Genotype 3** | |

Similarity coefficient = $\dfrac{2}{4}$ = 50%

**Similarity matrix**

| | Gen 1 | Gen 2 | Gen 3 |
|---|---|---|---|
| **Gen 1** | 100 | | |
| **Gen 2** | 66 | 100 | |
| **Gen 3** | 75 | 50 | 100 |

## Figure 3-B

[1]

| | Experiment 1 | | | | | Experiment 2 | | |
|---|---|---|---|---|---|---|---|---|
| | Gen 1 | Gen 2 | Gen 3 | | | Gen 1 | Gen 2 | Gen 3 |
| Gen 1 | 100 | | | | Gen 1 | 100 | | |
| Gen 2 | 66 | 100 | | | Gen 2 | 88 | 100 | |
| Gen 3 | 75 | 50 | 100 | | Gen 3 | 90 | 80 | 100 |

[2]

[3]

| | Gen1 | Gen2 | Gen3 |
|---|---|---|---|
| Gen1 | 100 | | |
| Gen2 | $\dfrac{(3\times 66)+(9\times 88)}{3+9}=83\%$ | 100 | |
| Gen3 | $\dfrac{(4\times 75)+(10\times 90)}{4+10}=86\%$ | $\dfrac{(4\times 50)+(10\times 80)}{4+10}=71\%$ | 100 |

## FIGURE 4

Consensus distance D

**FIGURE 5A**

## FIGURE 5B

16S rDNA identity

DNA hybridisation homology

Composite similarity matrix

## FIGURE 6

| Strain | Serotype | PFGE - *Xbal* | PFGE - *Avrll* |
|--------|----------|---------------|----------------|

## FIGURE 7

PFGE

Xbal

## FIGURE 8

## FIGURE 9

# FIGURE 10

# FIGURE 11

Pearson correlation (Opt0.30%) [3.6%-97.7%]
AFLP

| | | | |
|---|---|---|---|
| Xanthomonas | fragariae | | LMG 706 |
| Xanthomonas | fragariae | | LMG 708 |
| Xanthomonas | sacchari | | LMG 471 |
| Xanthomonas | sacchari | | LMG 476 |
| Xanthomonas | vesicatoria | | LMG 911 |
| Xanthomonas | vesicatoria | | LMG 920 |
| Xanthomonas | melonis | | LMG 8670 |
| Xanthomonas | melonis | | LMG 8672 |
| Xanthomonas | cassavae | | LMG 5264 |
| Xanthomonas | cassavae | | LMG 673 |
| Xanthomonas | codiaei | | LMG 8677 |
| Xanthomonas | codiaei | | LMG 8678 |
| Xanthomonas | vasicola | holcicola | LMG 736 |
| Xanthomonas | vasicola | holcicola | LMG 7416 |
| Xanthomonas | axonopodis | axonopodis | LMG 538 |
| Xanthomonas | oryzae | oryzae | LMG 5047 |
| Xanthomonas | oryzae | oryzicola | LMG 665 |
| Xanthomonas | hortorum | pelargonii | LMG 7314 |
| Xanthomonas | hortorum | hederae | LMG 733 |
| Xanthomonas | populi | | LMG 5743 |
| Xanthomonas | populi | | LMG 974 |
| Xanthomonas | axonopodis | dieffenbachiae | LMG 695 |
| Xanthomonas | axonopodis | begoniae | LMG 7303 |
| Xanthomonas | cucurbitae | | LMG 690 |
| Xanthomonas | cucurbitae | | LMG 8662 |
| Stenotrophomonas | maltophilia | | LMG 958 |
| Xanthomonas | translucens | poae | LMG 728 |
| Xanthomonas | translucens | translucens | LMG 876 |
| Xanthomonas | hyacinthi | | LMG 742 |
| Xanthomonas | hyacinthi | | LMG 8041 |

# FIGURE 12

DNA hybridization

| | | | |
|---|---|---|---|
| Xanthomonas | melonis | | LMG 8670 |
| Xanthomonas | melonis | | LMG 8672 |
| Xanthomonas | populi | | LMG 5743 |
| Xanthomonas | populi | | LMG 974 |
| Xanthomonas | cassavae | | LMG 5264 |
| Xanthomonas | cassavae | | LMG 673 |
| Xanthomonas | codiaei | | LMG 8677 |
| Xanthomonas | codiaei | | LMG 8678 |
| Xanthomonas | cucurbitae | | LMG 690 |
| Xanthomonas | cucurbitae | | LMG 8662 |
| Xanthomonas | oryzae | oryzae | LMG 5047 |
| Xanthomonas | oryzae | oryzicola | LMG 665 |
| Xanthomonas | vasicola | holcicola | LMG 736 |
| Xanthomonas | vasicola | holcicola | LMG 7416 |
| Xanthomonas | hortorum | pelargonii | LMG 7314 |
| Xanthomonas | hortorum | hederae | LMG 733 |
| Xanthomonas | axonopodis | dieffenbachiae | LMG 695 |
| Xanthomonas | axonopodis | begoniae | LMG 7303 |
| Xanthomonas | axonopodis | axonopodis | LMG 538 |
| Xanthomonas | fragariae | | LMG 706 |
| Xanthomonas | fragariae | | LMG 708 |
| Xanthomonas | vesicatoria | | LMG 911 |
| Xanthomonas | vesicatoria | | LMG 920 |
| Xanthomonas | translucens | poae | LMG 728 |
| Xanthomonas | translucens | translucens | LMG 876 |
| Xanthomonas | hyacinthi | | LMG 742 |
| Xanthomonas | hyacinthi | | LMG 8041 |
| Xanthomonas | sacchari | | LMG 471 |
| Xanthomonas | sacchari | | LMG 476 |
| Stenotrophomonas | maltophilia | | LMG 958 |

# FIGURE 13

Pairwise (OG:100% ,UG:0%) (FAST:2,10) Gapcost0% Disc. unk.
**16S rRNA**

| | | | |
|---|---|---|---|
| Xanthomonas | hyacinthi | | LMG 742 |
| Xanthomonas | hyacinthi | | LMG 8041 |
| Xanthomonas | translucens | poae | LMG 728 |
| Xanthomonas | translucens | translucens | LMG 876 |
| Xanthomonas | sacchari | | LMG 471 |
| Xanthomonas | sacchari | | LMG 476 |
| Xanthomonas | populi | | LMG 5743 |
| Xanthomonas | melonis | | LMG 8670 |
| Xanthomonas | cassavae | | LMG 673 |
| Xanthomonas | vesicatoria | | LMG 911 |
| Xanthomonas | cucurbitae | | LMG 690 |
| Xanthomonas | codiaei | | LMG 8677 |
| Xanthomonas | oryzae | oryzae | LMG 5047 |
| Xanthomonas | hortorum | pelargonii | LMG 7314 |
| Xanthomonas | vasicola | holcicola | LMG 736 |
| Xanthomonas | vasicola | holcicola | LMG 7416 |
| Xanthomonas | oryzae | oryzicola | LMG 665 |
| Xanthomonas | hortorum | hederae | LMG 733 |
| Xanthomonas | melonis | | LMG 8672 |
| Xanthomonas | codiaei | | LMG 8678 |
| Xanthomonas | fragariae | | LMG 708 |
| Xanthomonas | fragariae | | LMG 706 |
| Xanthomonas | cucurbitae | | LMG 8662 |
| Xanthomonas | cassavae | | LMG 5264 |
| Xanthomonas | vesicatoria | | LMG 920 |
| Xanthomonas | axonopodis | axonopodis | LMG 538 |
| Xanthomonas | axonopodis | dieffenbachiae | LMG 695 |
| Xanthomonas | axonopodis | begoniae | LMG 7303 |
| Xanthomonas | populi | | LMG 974 |
| Stenotrophomonas | maltophilia | | LMG 958 |

## FIGURE 14

AFLP+16S rRNA+DNA hybridization
AFLP+16S+DNA/DNA

| | | | |
|---|---|---|---|
| Xanthomonas | vesicatoria | | LMG 911 |
| Xanthomonas | vesicatoria | | LMG 920 |
| Xanthomonas | melonis | | LMG 8670 |
| Xanthomonas | melonis | | LMG 8672 |
| Xanthomonas | cassavae | | LMG 5264 |
| Xanthomonas | cassavae | | LMG 673 |
| Xanthomonas | codiaei | | LMG 8677 |
| Xanthomonas | codiaei | | LMG 8678 |
| Xanthomonas | vasicola | holcicola | LMG 736 |
| Xanthomonas | vasicola | holcicola | LMG 7416 |
| Xanthomonas | axonopodis | axonopodis | LMG 538 |
| Xanthomonas | oryzae | oryzae | LMG 5047 |
| Xanthomonas | oryzae | oryzicola | LMG 665 |
| Xanthomonas | hortorum | pelargonii | LMG 7314 |
| Xanthomonas | hortorum | hederae | LMG 733 |
| Xanthomonas | populi | | LMG 5743 |
| Xanthomonas | populi | | LMG 974 |
| Xanthomonas | fragariae | | LMG 706 |
| Xanthomonas | fragariae | | LMG 708 |
| Xanthomonas | axonopodis | dieffenbachiae | LMG 695 |
| Xanthomonas | axonopodis | begoniae | LMG 7303 |
| Xanthomonas | cucurbitae | | LMG 690 |
| Xanthomonas | cucurbitae | | LMG 8662 |
| Stenotrophomonas | maltophilia | | LMG 958 |
| Xanthomonas | translucens | poae | LMG 728 |
| Xanthomonas | translucens | translucens | LMG 876 |
| Xanthomonas | sacchari | | LMG 471 |
| Xanthomonas | sacchari | | LMG 476 |
| Xanthomonas | hyacinthi | | LMG 742 |
| Xanthomonas | hyacinthi | | LMG 8041 |

# FIGURE 15

AFLP +16S rRNA
**AFLP+16S+DNA/DNA**

| | | | |
|---|---|---|---|
| Xanthomonas | translucens | poae | LMG 728 |
| Xanthomonas | translucens | translucens | LMG 876 |
| Xanthomonas | hyacinthi | | LMG 742 |
| Xanthomonas | hyacinthi | | LMG 8041 |
| Xanthomonas | sacchari | | LMG 471 |
| Xanthomonas | sacchari | | LMG 476 |
| Xanthomonas | cassavae | | LMG 5264 |
| Xanthomonas | cassavae | | LMG 673 |
| Xanthomonas | codiaei | | LMG 8677 |
| Xanthomonas | codiaei | | LMG 8678 |
| Xanthomonas | cucurbitae | | LMG 690 |
| Xanthomonas | cucurbitae | | LMG 8662 |
| Xanthomonas | hortorum | pelargonii | LMG 7314 |
| Xanthomonas | hortorum | hederae | LMG 733 |
| Xanthomonas | vasicola | holcicola | LMG 736 |
| Xanthomonas | vasicola | holcicola | LMG 7416 |
| Xanthomonas | oryzae | oryzae | LMG 5047 |
| Xanthomonas | oryzae | oryzicola | LMG 665 |
| Xanthomonas | populi | | LMG 5743 |
| Xanthomonas | populi | | LMG 974 |
| Xanthomonas | melonis | | LMG 8670 |
| Xanthomonas | melonis | | LMG 8672 |
| Xanthomonas | vesicatoria | | LMG 911 |
| Xanthomonas | vesicatoria | | LMG 920 |
| Xanthomonas | fragariae | | LMG 706 |
| Xanthomonas | fragariae | | LMG 708 |
| Xanthomonas | axonopodis | axonopodis | LMG 538 |
| Xanthomonas | axonopodis | dieffenbachiae | LMG 695 |
| Xanthomonas | axonopodis | begoniae | LMG 7303 |
| Stenotrophomonas | maltophilia | | LMG 958 |

# FIGURE 16

Euclidian distance
**FAME**

| | | | |
|---|---|---|---|
| A | sylvestris | 52441 | Neutral |
| A | sylvestris | 52417 | Neutral |
| A | sylvestris | 52418 | Neutral |
| A | sylvestris | 52435 | Neutral |
| A | sylvestris | 52414 | Alkaline |
| A | sylvestris | 52434 | Alkaline |
| A | sylvestris | 52416 | Acid |
| A | sylvestris | 52412 | Acid |
| A | aberrans | 52449 | Neutral |
| A | aberrans | 52442 | Neutral |
| A | sp. | 52415 | Neutral |
| A | sp. | 52424 | Neutral |
| A | sp. | 52440 | Acid |
| A | aberrans | 52452 | Acid |
| A | aberrans | 52430 | Acid |

# FIGURE 17

Pairwise (OG:100%,UG:0%) (FAST:2,10) Gapcost:0%
**Histon H3**

| | | | |
|---|---|---|---|
| A | sylvestris | 52416 | Acid |
| A | sylvestris | 52414 | Alkaline |
| A | sylvestris | 52418 | Neutral |
| A | sylvestris | 52434 | Alkaline |
| A | sylvestris | 52412 | Acid |
| A | sylvestris | 52417 | Neutral |
| A | sylvestris | 52435 | Neutral |
| A | sylvestris | 52441 | Neutral |
| A | aberrans | 52449 | Neutral |
| A | aberrans | 52430 | Acid |
| A | aberrans | 52452 | Acid |
| A | aberrans | 52442 | Neutral |
| A | sp. | 52424 | Neutral |
| A | sp. | 52440 | Acid |
| A | sp. | 52415 | Neutral |

# FIGURE 18

FAME+Histon H3
**Composite**

| | | | | |
|---|---|---|---|---|
| A | sylvestris | 52418 | Neutral |
| A | sylvestris | 52435 | Neutral |
| A | sylvestris | 52417 | Neutral |
| A | sylvestris | 52414 | Alkaline |
| A | sylvestris | 52434 | Alkaline |
| A | sylvestris | 52416 | Acid |
| A | sylvestris | 52412 | Acid |
| A | sylvestris | 52441 | Neutral |
| A | aberrans | 52449 | Neutral |
| A | aberrans | 52442 | Neutral |
| A | aberrans | 52452 | Acid |
| A | aberrans | 52430 | Acid |
| A | sp. | 52415 | Neutral |
| A | sp. | 52424 | Neutral |
| A | sp. | 52440 | Acid |